# EUROPÄISCHE PATENTSCHRIFT

(11) **EP 4 213 755 B1**
(45) Veröffentlichungstag und Bekanntmachung des Hinweises auf die Patenterteilung: **17.04.2024**
(21) Anmeldenummer: 22708790.5
(22) Anmeldetag: 04.02.2022
(51) Int. Cl.: A61B 34/20, A61B 5/06, A61B 34/30, A61B 34/10, A61B 90/00, A61B 1/00, A61B 5/00

(54) **CHIRURGISCHES ASSISTENZSYSTEM**
SURGICAL ASSISTANCE SYSTEM
SYSTÈME D'ASSISTANCE CHIRURGICALE

(30) Priorität: 12.02.2021 DE 102021103411
(43) Veröffentlichungstag der Anmeldung: 26.07.2023
(73) Patentinhaber: B. Braun New Ventures GmbH, 79110 Freiburg im Breisgau (DE)
(72) Erfinder: SARVESTANI, Amir, 79104 Freiburg (DE); ZEPF, Rudolf, 78573 Wurmlingen (DE); KÜHN, Matthias, 79104 Freiburg (DE); LEMKE, Niels, 79312 Emmendingen (DE); SCHRÖER, Stefan, 79106 Freiburg (DE)
(74) Vertreter: Winter, Brandl - Partnerschaft mbB
(86) Internationale Anmeldenummer: PCT/EP2022/052730
(87) Internationale Veröffentlichungsnummer: WO 2022/171537

(56) Entgegenhaltungen:
- WO-A1-2014/049598
- WO-A1-2016/004007
- US-A1- 2006 281 971
- US-A1- 2015 313 634
- US-A1- 2019 008 592
- US-A1- 2019 192 232
- US-B2- 8 672 836

## Beschreibung

### Technisches Gebiet

Die vorliegende Offenbarung betrifft ein chirurgisches Assistenzsystem zur Verwendung bei einem chirurgischen Eingriff bei einem Patienten mit einer Darstellungsvorrichtung und einem Endoskop. Daneben betrifft die Offenbarung, ein computerlesbares Speichermedium für ein Aufnahmedarstellungsverfahren sowie einen Sterilraum gemäß den Oberbegriffen der nebengeordneten Ansprüche.

### Stand der Technik

Bei minimalinvasiven chirurgischen Eingriffen an weichem Gewebe, wie etwa der Bauchchirurgie, arbeiten chirurgische Assistenzsysteme mit (nur) computergestützter Navigation nach heutigem Stand der Technik noch nicht präzise genug. Aufgrund einer intraoperativen Bewegung des weichen Gewebes und des Fehlens einer Hartgewebereferenz, können präoperativ aufgenommene dreidimensionale 3D-Aufnahmedaten, wie etwa Computertomografie(CT-)Aufnahmedaten oder Magnetresonanztomografie(MRT-)Aufnahmedaten, während dem chirurgischen Eingriff mit dem Patienten nicht präzise genug verknüpft werden. Daher vertrauen Chirurgen weiterhin insbesondere auf endoskopische Aufnahmen, um ein Ziel-Operationsfeld zu finden, ein Gewebe zu identifizieren und eine Behandlung entsprechend durchzuführen.

Endoskopische Aufnahmen für sich allein sind jedoch hinsichtlich eines Informationsgehalts beschränkt und erlauben insbesondere keine Pfadplanung oder Gewebeidentifikation anhand präoperativer (dreidimensionaler) 3D-Aufnahmedaten. Dieser Mangel kann zu einer unbeabsichtigten Schädigung kritischer anatomischer Strukturen, wie etwa Nerven oder Blutgefäße führen, sowie auch zu einer Unter- oder Überresektion des anvisierten pathologischen Gewebes. Für das Organ der Leber beispielsweise wurden spezielle mathematische Modelle entwickelt, um intraoperative Deformationen auf Basis einer Oberflächenextraktion aus der endoskopischen Aufnahme zu korrigieren. Jedoch sind diese Technologien nicht zuverlässig genug und garantieren nicht die geforderte hohe Genauigkeit.

Allgemein profitieren Chirurgen also davon, wenn Ihnen sowohl intraoperative / intrakorporale endoskopische Aufnahmen als auch präoperative 3D-Aufnahmen zur Verfügung gestellt werden. Aktuell ist es jedoch nicht möglich, diese beide Modalitäten der Aufnahmen ohne zusätzliche Komplexität unter Einhaltung einer hohen Genauigkeitsanforderung miteinander zu verbinden.

Aus dem Stand der Technik sind chirurgische Assistenzsysteme für eine endoskopische Weichgewebenavigation bekannt, bei denen auf einem Display nebeneinander oder überlagert zwei unterschiedliche Darstellungen zweier Aufnahmen angezeigt werden. Neben einer ersten endoskopischen intrakorporalen Aufnahme in einem ersten Teilbereich des Displays wird eine dreidimensionale virtuelle CT-Aufnahme in einem zweiten Teilbereich des Displays statisch angezeigt. Jedoch muss ein Operateur bei dem chirurgischen Assistenzsystem nach Stand der Technik die virtuelle CT-Aufnahme händisch ändern, um die Darstellung der CT-Aufnahme nachzujustieren und an die aktuelle Ansicht des Endoskops mit der intrakorporalen Aufnahme anzupassen. Diese Vorgehensweise bindet Kapazitäten, ist hoch anspruchsvoll, wirkt ermüdend und garantiert nicht die notwendige Genauigkeit und damit Sicherheit bei einem chirurgischen Eingriff.

Ferner ist aus dem Stand der Technik eine navigationsbasierte Endoskopie bekannt, bei welcher ein Endoskop und ein Patient mittels einer Kamera nachverfolgt/getrackt werden. Sowohl an dem Endoskop als auch an dem Patienten sind hierfür Markierungselemente/Tracker angebracht, mit denen eine Registrierung durchgeführt wird. Auf diese Weise können CT-Aufnahmedaten mit der endoskopischen intrakorporalen Aufnahme korreliert werden. Jedoch erfordern ein solches System und Verfahren einen sehr hohen Zusatzaufwand.

Die US 2019/192232 A1 offenbart ein augmentiertes Navigationssystem für eine Visualisierung einer Position eines Instruments, wobei Lokalisierungssensoren an einer Kamera oder an einem Instrument angebracht sind.

Die US 8 672 836 B2 offenbart ein chirurgisches Navigationssystem im Bereich einer Bronchoskopie mit einem Endoskop für ein intrakorporales Video.

Die WO 2014/049598 A1, die US 2019/008592 A1, die US 20157313634 A1 und die WO 2016/004007 A1 offenbaren ebenfalls Navigationssysteme sowie Darstellungsmethoden.

Die US 2006/281971 A1 offenbart ein chirurgisches Navigationssystem mit einem Endoskop auf Basis von 3D-Aufnahmedaten eines Patienten. Insbesondere kommt eine VR-Brille als Anzeigevorrichtung zum Einsatz. Im Falle eines Fehlers einer Registrierung wird eine erneute Registrierung durchgeführt.

### Zusammenfassung der Offenbarung

Es ist daher die Aufgabe der vorliegenden Offenbarung, die Nachteile aus dem Stand der Technik zu vermeiden oder wenigstens zu mindern und insbesondere ein chirurgisches Assistenzsystem, ein (Aufnahme-)Darstellungsverfahren, ein Speichermedium sowie einen Sterilraum zur Verfügung zu stellen, welches/welcher OP Beteiligten, insbesondere einem (leitenden) Chirurgen, mit einem Blick eine intuitive und unterstützende Verschmelzung von Informationen einer intrakorporalen Aufnahme eines Endoskops sowie von digitalen 3D-Aufnahmedaten des Patienten bereitstellt, eine Hand-Augen-Koordination verbessert sowie einen sicheren Zugang zum Operationsfeld gewährleistet, um insbesondere Gewebeschäden durch unachtsame und unbeabsichtigte Handlungen zu vermeiden als auch eine Dauer eines chirurgischen Eingriffs zu minimieren. Insbesondere soll ein OP-Beteiligter von beiden Modalitäten, nämlich einer lokalen Navigation durch das Endoskop als auch der globalen Navigation durch die Aufnahmedaten des Patienten, in einfacher Weise profitieren und intuitiv und ermüdungsfrei einen chirurgischen Eingriff durchführen können.

Die Aufgabe und die Ziele der vorliegenden Offenbarung werden bei einem gattungsgemäßen chirurgischen Assistenzsystem durch die Merkmale des Anspruchs 1, bei einem gattungsgemäßen computerlesbaren Speichermedium für ein (Aufnahme-)Darstellungsverfahren durch die Merkmale des Anspruchs 9 und bei einem gattungsgemäßen Sterilraum durch die Merkmale des Anspruchs 11 erzielt.

Durch das chirurgische Assistenzsystem werden einem OP-Beteiligten, wie einem Chirurgen, intraoperativ beide (Aufnahme-)Modalitäten informationsadäquat und aufeinander abgestimmt bereitgestellt, indem die beiden (Aufnahme-)Modalitäten zueinander korreliert werden und der Chirurg die endoskopischen intrakorporalen Aufnahmen zur lokalen Führung und die (Ansichten der) 3D-Aufnahmedaten, insbesondere präoperative Aufnahmen, zur globalen Führung sowie für weiterführende Informationen zu etwa einem Gewebe nutzen kann.

Bei dem chirurgischen Assistenzsystem wird also initial die intrakorporale Aufnahme des Endoskops zu den digitalen 3D-Aufnahmedaten des Körpers des Patienten registriert, und zwar durch eine "aufnahmebasierte" Registrierung, indem in der intrakorporalen Aufnahme zumindest eine charakteristische Landmarke/ein Orientierungspunkt (in Aufnahmerichtung) und/oder anatomische Orientierung erfasst und bestimmt wird. Diese Landmarke und/oder Orientierung findet sich ebenfalls in den (virtuellen/digitalen) 3D-Aufnahmedaten wieder. Durch diesen zumindest einen Referenzpunkt (Landmarke) und/oder Orientierung kann die intrakorporale Aufnahme mit den 3D-Aufnahmedaten (geometrisch) korreliert werden. Insbesondere werden in der intrakorporalen Aufnahme mehrere charakteristische Landmarken, vorzugsweise zwei charakteristische Landmarken, besonders bevorzugt drei charakteristische Landmarken, erfasst und bestimmt.

Mit anderen Worten existieren bei dem chirurgischen Assistenzsystem zwei Seiten/Räume: ein erster, realer (geometrischer) Raum, mit dem realen Endoskop und dem realen Körper des Patienten sowie ein zweiter, virtueller (geometrischer) Raum mit einem virtuellen Endoskop und einem virtuellen Körper(-bereich) des Patienten. Ziel ist es, den realen Raum mit dem virtuellen Raum in einfacher Weise zu "verknüpfen", also zu korrelieren, so dass die geometrische Relation von Aufnahmekopf, insbesondere Endoskop, zu Körper in dem realen Raum der geometrischen Relation Aufnahmekopf, insbesondere Endoskop, zu Körper in dem virtuellen Raum entspricht. Dabei wird die Annahme getroffen, dass die digitalen 3D-Aufnahmedaten, zumindest teilbereichsweise, geometrisch gesehen dem realen Körper des Patienten entsprechen und näherungsweise eine 1:1-Abbildung in dem virtuellen Raum darstellen.

Wird nun bei einem chirurgischen Eingriff das Endoskop in den Körper des Patienten eingeführt, manuell oder robotergeführt, so erfasst dieses Endoskop über seinen Aufnahmekopf, insbesondere optisch, einen intrakorporalen Bereich des Körpers und fertigt eine intrakorporale Aufnahme, wie etwa ein zwei- oder ein dreidimensionales Bild an. Dieses aufgenommene Bild bzw. die aufgenommene anatomische Struktur findet sich, wie vorstehend erläutert, sowohl im realen Körper als auch im virtuellen, digitalen Körper wieder. Um nun eine initiale Registrierung zwischen dem realen Endoskop und dem realen Patienten (bzw. Körper des Patienten) vorzunehmen, wird sozusagen mittels der intrakorporalen Aufnahme eine Registrierung zwischen einem virtuellen Endoskop bzw. zumindest einem virtuellen Aufnahmekopf, und dem virtuellen Körper des Patienten vorgenommen und letztlich auf den realen Raum "übertragen". Hiernach sind der reale Raum und der virtuelle Raum sozusagen miteinander verknüpft und der virtuelle Raum kann gewissermaßen als "Abbildung" des realen Raums angesehen werden.

Die Registrierung kann insbesondere mittels folgender unterschiedlicher Registrierungsmethoden erfolgen:
- Punkt zu Punkt Registrierung, wobei zumindest drei Punkte für eine eindeutige Lageerkennung benötigt werden. Hierbei werden in der intrakorporalen Aufnahme zumindest drei zueinander beabstandete (anatomische) Punkte als Landmarken erfasst (diese spannen eine Ebene auf). Diese Erfassung kann etwa automatisch und/oder mit Hilfe einer manuellen Eingabe erfolgen. Es kann dann insbesondere die Steuereinheit dafür angepasst sein, in den 3D-Aufnahmedaten drei korrespondierende Punkte / Landmarken zu erfassen bzw. zu bestimmen und die Registrierung entsprechend durchzuführen;
- Punkt und Orientierung Registrierung, wobei zumindest ein Punkt als Landmarke und eine Orientierung benötigt werden. Hierbei wird über eine Referenz-Landmarke und einer Orientierung eine eineindeutige Festlegung erreicht; Der Begriff Orientierung bedeutet in diesem Fall, insbesondere gegenüber einem Referenzkoordinatensystem, dass drei Winkel für eine Festlegung der Orientierung angegeben werden, damit die Orientierung eindeutig ist.
- Oberflächenabgleich, wobei eine dreidimensional erfasste Oberfläche (3D-Oberfläche) der intrakorporalen Aufnahme (mit einer Vielzahl an Landmarken, welche die 3D-Oberfläche annähern) mit einer entsprechenden 3D-Struktur in den 3D-Aufnahmedaten, wie etwa einer segmentierten (dreidimensionalen) Struktur, korreliert und damit initial registriert wird. Beispielsweise kann eine (Teil-)Oberfläche einer Leber im realen Raum mit einer segmentierten 3D-Struktur der Leber in MRT-Aufnahmedaten korreliert werden.

Über die initiale Registrierung mittels der zumindest einen Landmarke und/oder Orientierung liegt zumindest initial die Relation von intrakorporaler Aufnahme zu den 3D-Aufnahmedaten (mit Bezug auf zumindest den Aufnahmekopf) vor.

Hiernach wird über das Trackingsystem eine Bewegung (veränderliche Lage über die Zeit) des realen Endoskops, insbesondere des Aufnahmekopfs, im realen Raum detektiert. Die Bewegung bzw. die Endoskop-Bewegungsdaten kann/können Translationen(Transformationen) und/oder Rotationen aufweisen, die sich auf bis zu sechs Freiheitsgrade aufsummieren können. Insbesondere ist die Transformation bzw. eine Transformationsmatrix von einem Handhabungsabschnitt, insbesondere dem Handstück, des Endoskops zu dessen Aufnahmekopf bekannt oder bestimmbar, so dass ausgehend von einer Bewegung des Handhabungsabschnitts, eine Bewegung des Aufnahmekopfs als auch umgekehrt, insbesondere durch die Steuereinheit, bestimmbar ist. Weiter bevorzugt kann auch eine Transformation bzw. Transformationsmatrix von dem Aufnahmekopf zu der intrakorporalen (Endoskop)Aufnahme und/oder eine Transformation von dem Handhabungsabschnitt zu der intrakorporalen Aufnahme bekannt oder bestimmbar sein, so dass von einer Bewegung der Intrakorporalen Aufnahme, eine Bewegung des Aufnahmekopfs bzw. des Handhabungsabschnitts und umgekehrt, insbesondere durch die Steuereinheit, bestimmbar ist. Es können also insbesondere Translationsbewegungen in drei Richtungen und Rotationsbewegungen um drei Achsen als Endoskop-Bewegungsdaten der Steuereinheit bereitgestellt werden. Die Steuereinheit überträgt die Endoskop-Bewegungsdaten des realen Raums auf die 3D-Aufnahmedaten, insbesondere auf den virtuellen Aufnahmekopf in dem virtuellen Raum. Man kann auch sagen, dass mit der Registrierung eine absolute Korrelation durchgeführt wird und mit der Erfassung einer Bewegung eine sich anschließende relative Korrelation.

Man kann auch sagen, dass die Endoskop-Bewegungsdaten eine Transformationsmatrix zwischen einem lokalen Koordinatensystem des Aufnahmekopfs zu einem lokalen Koordinatensystem des Körpers des Patienten (3D-Aufnahmedaten) im virtuellen Raum verändern und eine Ansicht der 3D-Aufnahmedaten entsprechend angepasst wird. Es sei angemerkt, dass von den bis zu sechs erfassten Freiheitsgraden der Endoskop-Bewegungsdaten insbesondere auch nur eine ausgewählte Menge als Endoskops-Bewegungsdaten in den virtuellen Raum weitergegeben werden können. Eine Teilmenge an Endoskop-Bewegungsdaten ist beispielsweise dann sinnvoll, wenn sich eine Position des Endoskops während einer Operation nur sehr wenig ändert, sondern in Hauptsache nur eine Orientierung.

Das chirurgische Assistenzsystem stellt mit der erzeugten Korrelationsdarstellung dem Chirurgen sowohl eine lokale als auch globale Navigationsmodalität zur Verfügung und verbessert eine Orientierung des Chirurgen. Der Chirurg ist mittels der durch etwa einen OP-Monitor ausgegebenen Korrelationsdarstellung in der Lage, hinter anatomische Strukturen im nativen Bild/der intrakorporalen Aufnahme "zu sehen". Der Chirurg kann besser und effizienter navigieren, um die gewünschte Region zu erreichen und weiß, wo er operieren muss. Auch wird eine Sicherheit während des chirurgischen Eingriffs erhöht und eine chirurgische Qualität verbessert. Eine bessere Orientierung führt zu kürzeren Eingriffszeiten. Insbesondere muss der Chirurg Schnittbilder nicht mehr im Kopf überlagern oder abschätzen, wo er sich denn gerade befindet. Dies führt zu einer Reduktion von Komplikationen und damit zu einer Reduktion von zusätzlichen Kosten und Risiken für Patient und Krankenhaus.

Mit anderen Worten wird die Aufgabe bei einem chirurgischen Assistenzsystem zur Verwendung bei einem chirurgischen Eingriff bei einem Patienten erzielt, indem dieses Assistenzsystem aufweist: eine Darstellungsvorrichtung, insbesondere einen OP-Monitor, zur Darstellung eines visuellen Inhalts, insbesondere eines Bildes, ein Endoskop, insbesondere ein Videoendoskop, mit einem distalen bildgebenden Aufnahmekopf, welches dafür vorgesehen und angepasst ist, eine intrakorporale Aufnahme des Patienten zu erstellen und diese intrakorporale Aufnahme digital/computerlesbar bereitzustellen, eine Datenbereitstellungseinheit, insbesondere eine Speichereinheit, welche dafür vorgesehen und angepasst ist, digitale 3D-Aufnahmedaten, insbesondere präoperative 3D-Aufnahmedaten, eines Patienten digital/computerlesbar bereitzustellen, insbesondere aufzubereiten und bereitzustellen, und eine Steuereinheit/Berechnungseinheit/Computereinheit, die dafür angepasst ist, die intrakorporale Aufnahme des Endoskops sowie die 3D-Aufnahmedaten zu verarbeiten. Das chirurgische Assistenzsystem weist ferner auf: eine Registrierungseinheit, die dafür vorgesehen und angepasst ist, in der intrakorporalen Aufnahme des Endoskops zumindest eine insbesondere mehrere, bevorzugt zwei, besonders bevorzugt drei, insbesondere vorbestimmte, anatomischen Landmarke und/oder eine anatomische Orientierung zu erfassen sowie zumindest eine korrespondierende anatomische Landmarke und/oder eine Orientierung in den bereitgestellten 3D-Aufnahmedaten zu bestimmen und über die korrespondierenden anatomischen Landmarken und/oder Orientierung die intrakorporale Aufnahme mit den 3D-Aufnahmedaten, zumindest initial, zu registrieren und das Endoskop, insbesondere den Aufnahmekopf, relativ zu dem (Körper des) Patienten zu registrieren; ein Trackingsystem, welches dafür vorgesehen und angepasst ist, kontinuierlich/laufend eine Lage des Endoskops und/oder eine Bewegung des Endoskops, insbesondere des Aufnahmekopfs, zu erfassen und der Steuereinheit als Endoskop-Bewegungsdaten bereitzustellen. Die Steuereinheit ist ferner dafür angepasst, eine Korrelationsdarstellung mit sowohl (mindestens) einer Darstellung der intrakorporalen Aufnahme als auch (mindestens) einer Darstellung einer Ansicht der 3D-Aufnahmedaten zu erzeugen, in welcher die intrakorporale Aufnahme und die Ansicht(en) der 3D-Aufnahmedaten hinsichtlich des Endoskops, insbesondere des Aufnahmekopfs, zueinander korreliert sind. Hierbei werden die erfassten Endoskop-Bewegungsdaten auf zumindest eine virtuelle Position und/oder Orientierung eines virtuellen Aufnahmekopfs, insbesondere eines virtuellen Endoskops, in der Ansicht der 3D-Aufnahmedaten für eine korrelierte Bewegung übertragen, und die Steuereinheit ist dafür angepasst die so erzeugte Korrelationsdarstellung durch die Darstellungsvorrichtung visuell auszugeben. Die Steuereinheit kann beispielsweise einen Prozessor (CPU) für eine entsprechende Verarbeitung und Steuerung aufweisen. Die Datenbereitstellungseinheit kann die Daten auch aufbereiten, wenn sich kontextbezogen Änderungen des Modells ergeben (z.B. durch Deformation oder Abtrennen von Gewebe.

Grundsätzlich sieht die vorliegende Offenbarung bei einem chirurgischen Assistenzsystem also eine erste (reale) Aufnahme, nämlich die intrakorporale Aufnahme durch das Endoskop, sowie eine zweite (virtuelle) Aufnahme in Form einer (vorbestimmten) Ansicht von 3D-Aufnahmedaten, wie etwa CT-Aufnahmedaten oder MRT-Aufnahmedaten, vor. Beide Aufnahmen werden einem OP-Beteiligten wie dem Chirurgen auf insbesondere einem OP-Monitor visuell angezeigt. Konkret wird insbesondere in einem (Teil-)Bereich der Korrelationsdarstellung und damit des OP-Monitors die zeitaktuelle reale Endoskop-Videoaufnahme als intrakorporale Aufnahme dargestellt, während in einem anderen (Teil-)Bereich des OP-Monitors eine (ausgewählte) korrelierte Ansicht der 3D-Aufnahmedaten dargestellt wird. Insbesondere sind beide Darstellungen nebeneinander angeordnet. Alternativ können auch beide Darstellungen überlagert angezeigt werden (Augmented Reality). Dadurch wird ein Chirurg in die Lage versetzt mittels der Ansicht der 3D-Aufnahmedaten eine globale Navigation durchzuführen, um zu einem Ziel-Operationsbereich zu gelangen, während die endoskopische intrakorporale Aufnahme detaillierte Bilder zu etwa einer tatsächlichen aktuellen Lage eines Weichteils liefert. Da beide Aufnahmen zueinander korreliert sind, muss der Chirurg keine manuellen Anpassungen oder virtuelle Drehungen bzw. Verschiebungen der Aufnahmen zueinander vornehmen. Die Korrelation der beiden Aufnahmen erfolgt insbesondere automatisiert durch die chirurgische Assistenzvorrichtung.

Mit noch anderen Worten betrifft die vorliegende Offenbarung ein intraoperatives chirurgisches Assistenzsystem, welches ein endoskopisches Bild (intrakorporale Aufnahme) und ein, insbesondere präoperatives, 3D-Bild (3D-Aufnahmedaten) des Patienten, insbesondere nebeneinander, anzeigt. Beide Bilder (Aufnahmen) sind korreliert und beziehen sich auf die aktuelle Position und/oder Orientierung des Aufnahmekopfs, insbesondere des Endoskops. Vorzugsweise werden bei einem Einführen des Endoskops in ein Operationsfeld insbesondere vordefinierte anatomische Landmarken und/oder Orientierungen vom Chirurgen anhand des endoskopischen Bildes (intrakorporalen Aufnahme) ausgewählt und mit entsprechenden anatomischen Landmarken und/oder Orientierungen in den 3D-Datensätzen (3D-Aufnahmedaten) korreliert, wobei dieser Schritt als Registrierung bezeichnet wird. Es sind verschiedene Registrierungsmethoden möglich:
- Punkt zu Punkt, mindestens 3 Punkte
- Punkt und Orientierung
- Oberflächenabgleich, Korrelieren der 3D-Oberfläche aus dem Endoskopbild (intrakorporalen Aufnahme) mit einer, insbesondere entsprechenden segmentierten, Struktur im 3D-Bild (3D-Aufnahmedaten).

Eine Registrierung mittels einer Landmarke hat zwar den Vorteil, dass auch ein Abstand zu einem Objekt, etwa einer Leber, mit aufgenommen wird und so die absoluten Dimensionen von intrakorporaler Aufnahme und 3D-Aufnahmedaten "gematcht" werden können, jedoch kann es auch schon ausreichend sein, wenn lediglich das Objekt richtig gedreht wird (nur Winkelanpassung). Dies kann gerade bei segmentierten Darstellungen sinnvoll sein, da die 3D-Aufnahmedaten insbesondere auch nur ein Organ umfassen können.

Der Begriff 3D definiert, dass die Aufnahmedaten des Patienten räumlich, also dreidimensional vorliegen. Der Körper des Patienten oder zumindest ein Teilbereich des Körpers mit räumlicher Ausdehnung kann in einem dreidimensionalen Raum mit etwa einem kartesischen Koordinatensystem (X, Y, Z) digital als Aufnahmedaten vorliegen.

Der Begriff Lage definiert sowohl eine Position als auch eine Orientierung. Eine Position kann etwa in einem kartesischen Koordinatensystem mit (X, Y, Z) angegeben werden und die Orientierung mit drei Winkeln (α, β, γ).

Der Begriff Ansicht beschreibt, insbesondere in den 3D-Aufnahmedaten, insbesondere an einem (vorbestimmbaren) Punkt eine (vorbestimmbare) Blickrichtung/Perspektive mit insbesondere (vorbestimmbarer) Rotation, ähnlich einer auswählbaren Ansicht bei einem CAD-Modell. Es wird gewissermaßen eine Lage eines Blickpunktes definiert.

Der Begriff real definiert die "Seite" des physischen (realen) Endoskops und Patienten, bei dem der tatsächliche Eingriff vorgenommen wird, wohingegen virtuell die "Seite" des digitalen, virtuellen Endoskops und der aufgenommenen virtuellen Patientendaten definiert, welche computerlesbar vorliegen und die in einfacher Weise digital manipulierbar sind, um etwa individuelle Informationen anzeigen zu lassen.

In einer Alternative kann das Trackingsystem als ein endoskopinternes Trackingsystem ausgebildet sein und das Endoskop, insbesondere ein Handhabungsabschnitt und/oder der Aufnahmekopf, einen internen Bewegungssensor, vorzugsweise einen Beschleunigungssensor und/oder einen Drehratensensor, insbesondere eine inertiale Messeinheit (IMU; inertial measurement unit), zur Erfassung der Endoskop-Bewegungsdaten aufweisen. So benötigt das chirurgische Assistenzsystem keine weiteren externen Systeme oder Messvorrichtungen, sondern es reicht allein ein im Endoskop integrierter Sensor, der räumlich klein ist. Über den internen Bewegungssensor kann sozusagen autark eine Bewegung des Endoskops, insbesondere des Aufnahmekopfs, detektiert werden und digital als Endoskop-Bewegungsdaten der Steuereinheit bereitgestellt werden. Mit anderen Worten kann zur Bestimmung der Bewegung des Endoskops, insbesondere des Aufnahmekopfs, das Endoskop zumindest einen Bewegungssensor aufweisen, vorzugsweise interne Sensoren wie insbesondere eine IMU.

Gemäß einer weiteren Alternative kann, alternativ oder zusätzlich zu dem endoskopinternen Trackingsystem, für eine Ausbildung des Trackingsystems, die Steuereinheit dafür angepasst sein, über eine Bildanalyse /Datenauswertung von sich bewegenden anatomischen Strukturen in der intrakorporalen Aufnahme die Bewegung des Endoskops zu bestimmen und als Endoskop-Bewegungsdaten bereitzustellen. Über geeignete Bildanalysemethoden kann über eine Änderung des Bildes auf eine Bewegung geschlossen werden. Dies kann etwa mit einer Videoaufnahme einer Frontkamera eines Autos verglichen werden, bei der anhand von Fahrbahnmarkierungen oder etwa eines sich bewegenden Horizonts eine Bewegung detektiert wird. Das Trackingsystem in Form der angepassten Steuereinheit berechnet also anhand der zeitlich veränderlichen intrakorporalen Aufnahme eine Bewegung, insbesondere des Aufnahmekopfs. Insbesondere sind hierfür die optischen Parameter der endoskopischen intrakorporalen Aufnahme bekannt und/oder der Aufnahmekopf, insbesondere die endoskopische Kamera des Aufnahmekopfs, ist kalibriert. Mit anderen Worten können Bewegungen des Endoskops auch über das endoskopische Bild / die intrakorporale Aufnahme erkannt werden, indem analysiert wird, in welche Richtung und insbesondere mit welcher Geschwindigkeit sich die (anatomischen) Strukturen im Bild bewegen. Ein Vorteil einer solchen Bildanalyse ist etwa, dass auch selbst ohne Bewegungssensoren die Endoskop-Bewegungsdaten ermittelt werden können.

Vorteilhafte Ausführungen sind in den Unteransprüchen beansprucht und werden insbesondere nachfolgend erläutert.

Gemäß einer weiteren Ausführungsform können alternativ oder zusätzlich zu endoskopinternen Sensoren für das Trackingsystem auch externe Sensoren oder Referenzmarken vorgesehen werden, die von einem Kamerasystem erfasst werden. Insbesondere kann das Trackingsystem ein Kamerasystem aufweisen, welches das Endoskop, insbesondere einen Handhabungsabschnitt, (laufend) räumlich erfasst, dessen Lage bestimmt und an die Steuereinheit als Endoskop-Bewegungsdaten bereitstellt. Hierüber kann durch eine vorbestimmte geometrische Relation auf die Lage des Aufnahmekopfs geschlossen werden. Mit anderen Worten kann das Trackingsystem auch eine externe Kamera, insbesondere eine 3D-Kamera, aufweisen, das einen Eingriffsbereich erfasst und bei einem starren Endoskop laufend eine Lage des Betätigungsabschnitts detektiert und anhand des detektierten Betätigungsabschnitt zeitlich die Lage und damit die Bewegung des Endoskops, insbesondere des Aufnahmekopfs, bestimmt.

Insbesondere kann das Trackingsystem sowohl den in dem Endoskop vorgesehenen Bewegungssensor, insbesondere den Beschleunigungssensor und/oder Drehratensensor, besonders bevorzugt die inertiale Messeinheit, aufweisen als auch die Steuereinheit dafür angepasst sein, über die Bildanalyse die Endoskop-Bewegungsdaten zu erfassen, wobei aus den zumindest zwei Ergebnissen der Endoskop-Bewegungsdaten ein kombinierter Wert der beiden Ergebnisse, insbesondere ein angepasster Mittelwert oder ein gewichteter Wert der Endoskop-Bewegungsdaten, als Endoskop-Bewegungsdaten (als finales Ergebnis) berechnet wird. Die Gewichtung kann für jedes einzelne Element/Komponente der Rotation und Translation (sechs Elemente/Freiheitsgrade) vorgenommen werden. Beispielsweise können inertiale Messeinheiten (IMU) Rotationen genauer bestimmen als Translationen. Insbesondere werden aus den zumindest zwei Ergebnissen über die inertiale Messeinheit (IMU) die Rotationsdaten und über die Bildanalyse die Translationsdaten für die Endoskop-Bewegungsdaten bereitgestellt. Insbesondere kann die Gewichtung so erfolgen, dass in dem Ergebnis der IMU die Rotations-Komponenten mit mehr als 80%, insbesondere 100%, gewichtet werden, wohingegen die Translations-Komponenten mit weniger als 20%, insbesondere 0% gewichtet werden, wobei in dem Ergebnis der Bildanalyse die Rotations-Komponenten komplementär mit weniger als 20%, insbesondere 0%, gewichtet werden und die Translations-Komponenten mit mehr als 80%, insbesondere 100%. Der Mittelwert kann etwa ein arithmetischer Mittelwert, ein geometrischer Mittelwert oder ein quadratischer Mittelwert sein, der insbesondere bei jeder einzelnen Komponente gebildet wird. Auf diese Weise werden (zumindest) zwei unterschiedliche "Sensorsysteme" miteinander verbunden. Durch eine redundante Erfassung mit Kombination, insbesondere Mittewertbildung oder Gewichtung, können systembedingte Ungenauigkeitsfehler weiter minimiert und eine Genauigkeit erhöht werden. Insbesondere können also die internen Sensoren und die Bildanalyse kombiniert werden, um die Bewegung des Endoskops zu bestimmen. Als Ergebnis kann der Chirurg in den 3D-Bildern/3D-Aufnahmedaten die tatsächliche Position des Endoskops, insbesondere des Aufnahmekopfs, verfolgen und so kritische Strukturen identifizieren oder einen Weg aus einem präoperativen Plan verfolgen.

Gemäß einer weiteren bevorzugten Ausführungsform kann die Steuereinheit dafür angepasst sein, insbesondere auf Basis einer in der Speichereinheit hinterlegten Datenbank mit geometrisch vordefinierten Strukturen medizinischer Instrumenten, in der intrakorporalen Aufnahme zumindest ein (hinterlegtes) medizinisches Instrument lagekorrekt zu erkennen und das erkannte medizinische Instrument in der Ansicht der 3D-Aufnahmedaten als virtuelles medizinisches Instrument lagekorrekt, insbesondere korreliert, anzuzeigen, so dass das medizinische Instrument in beiden Aufnahmen angezeigt wird. Der Begriff lagekorrekt definiert die Kombination aus positionskorrekt und orientierungskorrekt, also an der übereinstimmenden Position und Lage. Mit anderen Worten kann also insbesondere auch zumindest ein Instrument in beiden Aufnahmen dargestellt werden, so dass der Chirurg nicht nur etwa die Position und/oder Orientierung des Endoskops in den 3D-Aufnahmedaten, sondern auch die Position und/oder Orientierung, des medizinischen Instruments visualisieren kann. Dazu werden die medizinischen Instrumente in der intrakorporalen Aufnahme detektiert und in den 3D-Aufnahmedaten (3D-Bild) an ihrer (tatsächlichen) Position virtuell überlagert. Voraussetzung hierfür ist, dass die Instrumente im endoskopischen Bild sichtbar sind und ihre geometrische Form bekannt ist.

Insbesondere können in einer in der Speichereinheit hinterlegten Datenbank auch Daten zu dem Aufnahmekopf, insbesondere ein Kamerakopftyp, und/oder Daten zum Endoskop, insbesondere ein integriertes Endoskop und/oder eine Art "Dummyendoskop" für einen Kamerakopf, gespeichert sein. Beispielsweise können hinterlegte geometrischen Daten des Aufnahmekopfs in der Korrelationsdarstellung eingeblendet entsprechend werden.

Insbesondere kann die Steuereinheit dafür angepasst sein, anhand von der Position und/oder Orientierung des Endoskops, insbesondere des Aufnahmekopfs, den in der Speichereinheit hinterlegten Daten zu dem Endoskop, insbesondere zu optischen Parametern des Aufnahmekopfs, und vorzugsweise einem Abstand zu den anvisierten anatomischen Strukturen, einen Sichtkegel des Aufnahmekopfs zu bestimmen und in der Korrelationsdarstellung in der Darstellung der 3D-Aufnahmedaten einen virtuellen Sichtkegel des virtuellen Aufnahmekopfs mit einzublenden. Auf diese Weise sieht der Anwender, welchen Bereich er auf dem 3D-Aufnahmedaten gerade anschaut. Das ist beispielsweise dann vorteilhaft, wenn die Maßstäbe nicht gleich sind und etwa die 3D-Aufnahmedaten herausgezoomt dargestellt sind. Der Begriff Sichtkegel beschreibt ein dreidimensionales Volumen, das durch ein durch eine Optik definiertem Sichtfeld und entsprechendem Abstand zu einer Front gebildet ist.

Vorzugsweise kann die Registrierungseinheit über die initiale Registrierung hinaus zu zumindest einem weiteren, insbesondere vorbestimmten, Zeitpunkt eine erneute Registrierung durchführen, um eine Genauigkeit der Korrelation weiter zu erhöhen. Da ausgehend von einer registrierten (absoluten) Startposition des Aufnahmekopfs die Endoskop-Bewegungsdaten relative Daten sind (ohne externen absoluten Bezug), kann eine erneute Registrierung (als absolute Korrelation) die Genauigkeit weiter erhöhen. Insbesondere kann die nachfolgende Registrierung nach einer vorbestimmten Zeit oder nach einer vorbestimmten Summe von Bewegungen durchgeführt werden. Insbesondere können auch mehrere sich wiederholende Registrierungen nach jeweils einer vorbestimmten Zeit, einem vorbestimmten Weg oder einer vorbestimmten Bewegungssumme als "Reset" durchgeführt werden. Eine Neu-Registrierung zwischen der intrakorporalen Aufnahme des Endoskops und den 3D-Aufnahmedaten (3D-Modell/3D-Bild) kann also jederzeit erneut durchgeführt werden, um die Genauigkeit im Falle von Weichteilbewegungen weiter zu verbessern.

In einer bevorzugten Ausführungsform kann das Endoskop als ein starres Videoendoskop ausgebildet sein. Bei einem starren Endoskop liegt eine konstante geometrische Relation zwischen einem Handhabungsabschnitt und dem Aufnahmekopf vor. So kann beispielsweise ein Bewegungssensor in einem Handstück vorgesehen sein, der die Bewegung in dem Handstück registriert und die Steuereinheit kann dafür angepasst sein anhand der erfassten Bewegungsdaten und der geometrischen Relation auf die Bewegung des Aufnahmekopfs zu schließen.

Vorzugsweise kann die Steuereinheit dafür angepasst sein, in der Korrelationsdarstellung in der Darstellung der Ansicht der 3D-Aufnahmedaten und/oder der Darstellung der intrakorporalen Aufnahme zusätzlich zu dem realen und/oder virtuellen Endoskop, in einer Speichereinheit hinterlegte vorgeplante medizinische Instrumente und/oder Implantate, insbesondere einen vorgeplanten Trokar, lagekorrekt (virtuell) anzuzeigen. Es kann also insbesondere ein vorgeplanter Trokar in den 3D-Aufnahmedaten bzw. in der Ansicht der 3D-Aufnahmedaten relativ zu der aktuellen Position des Endoskops angezeigt werden.

Gemäß einer weiteren bevorzugten Ausführungsform kann die Steuereinheit dafür angepasst sein, in der Korrelationsdarstellung in Darstellung der Ansicht der 3D-Aufnahmedaten und/oder der intrakorporalen Aufnahme einen geplanten Pfad/eine Trajektorie anzuzeigen, um einem Chirurgen einen Weg zum Operationsfeld zu weisen. Hierdurch wird eine Orientierung des Chirurgen weiter verbessert. Eine Trajektorie kann insbesondere durch einen eingeblendeten Pfeil mit Pfeilspitze in Richtung des geplanten Pfades angegeben werden, der sich bei einer Bewegung des Endoskops anpasst und stets in Richtung des Pfades zeigt. Alternativ oder zusätzlich kann ein geplanter Pfad auch etwa in Form einer dreidimensionalen Linie entlang des Pfades in den 3D-Aufnahmedaten eingeblendet werden. Alternativ oder zusätzlich können auch prä-operativ geplante Annotationspunkte in den 3D-Aufnahmedaten intraoperativ eingeblendet werden, um sich etwa einem Zielgebiet zu nähern oder kritische Strukturen zu erkennen.

Vorzugsweise kann die Steuereinheit dafür angepasst sein, die Ansicht der 3D-Aufnahmedaten als 3D-Szene/3D-Rendering und/oder als zweidimensionale Querschnitte relativ zu einem Bild-Koordinatensystem des Endoskops und/oder entlang einer Aufnahmeachse des Endoskops, insbesondere des Aufnahmekopfs, und/oder als virtuelles Endoskopbild zu erstellen. Die 3D-Aufnahmedaten können also etwa ein gerendertes 3D-Modell, mit oder ohne segmentierten anatomischen Strukturen, sein, welches in dem virtuellen Raum frei bewegbar und von unterschiedlichen Positionen und Ansichten aus unterschiedlichen Betrachtungswinkeln mit insbesondere unterschiedlichen Transparenzen von unterschiedlichen anatomischen Strukturen darstellbar ist, ähnlich eines CAD-Modells. Die Steuereinheit erstellt dann eine ausgewählte Ansicht der 3D-Szene. Auch können beispielsweise Mengen an anatomischen Strukturen im virtuellen Raum ausgeblendet werden, um eine noch bessere Ansicht der relevanten Strukturen zu erhalten. Alternativ oder zusätzlich können die 3D-Aufnahmedaten auch als (segmentierte) zweidimensionale Querschnitte relativ zu einem Koordinatensystem des Endoskops, insbesondere einem Bild-Koordinatensystem des Endoskops, und/oder entlang einer Achse vorliegen, wie etwa bei MRT- oder CT-Aufnahmedaten. Das Bild-Koordinatensystem entspricht dem (lokalen) Koordinatensystem der intrakorporale Aufnahme. Alternativ können auch zweidimensionale Querschnitte relativ zu einem lokalen Koordinatensystem des Aufnahmekopfs erstellt werden. Das Bild-Koordinatensystem der intrakorporalen Aufnahme kann über eine Transformation zu dem lokalen Koordinatensystem des Aufnahmekopfs und weiter über eine Transformation zu einem lokalen Koordinatensystem eines Handstücks auch zurückberechnet werden. Die Steuereinheit kann dann etwa, ähnlich wie bei Ultraschallbilder, die Ansicht der 3D-Aufnahmedaten entlang der Achse der Aufnahmerichtung bzw. des Aufnahmekopfs, insbesondere des Endoskops, korrespondierend zu der Position des Aufnahmekopfs insbesondere des Endoskops, erstellen. Dies ermöglicht beispielsweise anatomische Strukturen hinter dem vom Endoskop aufgenommenen Bild zu erkennen. Alternativ oder zusätzlich zu den Querschnitten entlang der Aufnahmeachse können auch diagonale oder individuelle von etwa der Anatomie abhängige oder vordefinierte Schnittdarstellungen erstellt werden. Vorzugsweise kann gegenüber einem Referenzkoordinatensystem des Endoskops (mit X-Y-Z Achsen) betrachtete X-Schnitte und/oder Y-Schnitte und/oder Z-Schnitte erstellt werden. Bei Z-Schnitten (also den Schnitten parallel zum Sichtfeld des Endoskops und senkrecht auf eine lokale Z-Achse des Referenzkoordinatensystems des Endoskops) kann insbesondere der Schnitt über eine Vor- und Rückwärtsbewegung des Endoskops entsprechend verschoben werden. Ein Bewegungsbereich zu einem Objekt hin kann etwa durch einen Arbeitsabstand limitiert sein. Eine Z-Achse liegt bei einem starren Endoskop insbesondere koaxial zu dessen Längsachse und die Z-Schnitte sind entsprechend orthogonal zu der Z-Achse und damit parallel zu dem Sichtfeld angeordnet. Alternativ oder zusätzlich kann die Steuereinheit die Ansicht der 3D-Aufnahmedaten als virtuelles Endoskopbild/Endoskopperspektive erstellen, welches die reale Aufnahme des Aufnahmekopfs imitiert. Mit anderen Worten wird eine Ansicht der 3D-Aufnahmedaten erstellt, in welcher der virtuelle Aufnahmekopf des virtuellen Endoskops die gleiche Lage (also Position und Orientierung) wie der reale Aufnahmekopf aufweist, und eine virtuelle Aufnahme mit genau den optischen Parametern wie des reale Endoskops erstellt, so dass die reale intrakorporale Aufnahme sozusagen als virtuelle intrakorporale Aufnahme im virtuellen Raum dargestellt wird (1:1 Darstellung reale Aufnahme-virtuelle Aufnahme). Hierbei "sieht" der Chirurg sowohl in der realen als auch der virtuellen Darstellungen in genau "dieselbe Richtung an derselben Position". Mit anderen Worten können die 3D-Aufnahmedaten, insbesondere die Ansicht der 3D-Aufnahmedaten (3D-Bild) als 3D-Szene/3D-Rendering dargestellt werden, mit oder ohne segmentierte anatomische Strukturen. Alternativ oder zusätzlich kann das 3D-Bild als 2D-Querschnitte entlang der Achse des Endoskops dargestellt werden, ähnlich wie bei Ultraschallbildern. Die 3D-Bilder können alternativ oder zusätzlich als virtuelles endoskopisches Bild dargestellt werden, das die Blickrichtung des Endoskops nachahmt, ähnlich eines Durchflugmodells.

Vorzugsweise weist das chirurgische Assistenzsystem eine Ansichtenauswahleinheit auf, in der eine vordefinierten Anzahl unterschiedlicher Ansichten hinterlegt ist. Insbesondere ist eine Vogelperspektive, eine Endoskopsansicht (in Aufnahmerichtung), eine Seitenansicht und eine Unteransicht gespeichert. Bei einer Auswahl einer Ansicht, insbesondere durch den Chirurgen, passt die Steuereinheit die Ansicht der 3D-Aufnahmedaten an die ausgewählte Ansicht entsprechend an. Mit anderen Worten kann das chirurgische Assistenzsystem vorkonfigurierte Einstellungen aufweisen, die es dem Chirurgen erlauben, verschiedene Ansichten der 3D-Aufnahmedaten (Darstellungen des 3D-Bildes) wie etwa Vogelperspektive, Endoskopansicht, Seitenansichten, Bodenansicht, für die zu untersuchende Region zu übernehmen, um unterschiedliche Perspektiven einzunehmen und die individuell verfügbaren und unterschiedlichen Inhalte für Entscheidungen des Chirurgen zu berücksichtigen.

Insbesondere führt die Steuereinheit die Funktion des Trackingsystems aus.

Gemäß einer Ausführungsform kann das chirurgische Assistenzsystem ferner einen Roboter aufweisen und das Endoskop, insbesondere ein Handhabungsabschnitt des Endoskops, robotergeführt sein, insbesondere mittels eines Roboterarms, und das Trackingsystem kann die Lage und/oder Orientierung und/oder Bewegung des Endoskops, insbesondere des Aufnahmekopfs, über eine zugehörige Lage und/oder Orientierung und/oder Bewegung des Roboters, insbesondere des Roboterarms, bestimmen. Im Falle eines roboterassistierten chirurgischen Eingriffs, bei dem das Endoskop über den Roboter geführt wird, kann vorzugsweise eine Position und/oder Orientierung und über die Zeit gesehen damit eine Bewegung des Endoskops, insbesondere des Aufnahmekopfs, direkt von einem Achssystem oder Steuerbefehlen mit entsprechender Transformation berechnet werden. Die Detektion der Position und/oder Orientierung und/oder Bewegung findet somit nicht im Endoskop selbst, sondern im Roboter statt. Mit anderen Worten wird bei einem Robotik-System die Bewegung und/oder Position und/oder Orientierung des Endoskops von dem Roboter, insbesondere dem Roboterarm, abgeleitet.

Hinsichtlich eines Aufnahmedarstellungsverfahren zur korrelierten Darstellung von zwei unterschiedlichen Aufnahmen werden die Aufgaben und Ziele der vorliegenden Offenbarung durch die Schritte gelöst: Einlesen von digitalen 3D-Aufnahmedaten, insbesondere von prä-operativen 3D-Aufnahmedaten, eines Patienten; Erstellen einer intrakorporalen Aufnahme durch ein Endoskop; Erfassen von zumindest einer Landmarke und/oder Orientierung in der intrakorporalen Aufnahme; Bestimmen einer korrespondierenden Landmarke und/oder Orientierung in den 3D-Aufnahmedaten; Registrieren der 3D-Aufnahmedaten zu der intrakorporalen Aufnahme über die zumindest eine erfasste Landmarke und/oder Orientierung; Erzeugen von zumindest einer Korrelationsdarstellung mit einer Darstellung der intrakorporalen Aufnahme und einer Darstellung einer Ansicht der 3D-Aufnahmedaten sowie vorzugsweise Ausgeben der Korrelationsdarstellung durch die Darstellungsvorrichtung; Kontinuierliches Erfassen einer Lage und/oder Orientierung und/oder einer Bewegung des Endoskops, insbesondere des Aufnahmekopfs; Übertragen der erfassten Bewegung des Endoskops, insbesondere des Aufnahmekopfs, auf zumindest eine virtuelle Position und/oder Orientierung eines virtuellen Aufnahmekopfs, insbesondere eines virtuellen Endoskops, in der Ansicht der 3D-Aufnahmedaten und Erzeugung einer aktualisierten Korrelationsdarstellung mit der intrakorporalen Aufnahme und der aktualisierten Ansicht der 3D-Aufnahmedaten und Ausgeben der Korrelationsdarstellung durch die Darstellungsvorrichtung.

Gemäß einer ggf. unabhängig beanspruchbaren Ausführungsform kann vorzugsweise das Aufnahmedarstellungsverfahren die folgenden Schritte aufweisen: Erfassen 3D-Aufnahme(-daten), insbesondere präoperative 3D-Aufnahme(-daten); Vorzugsweise Segmentieren 3D-Aufnahme(-daten); Bereitstellen der 3D-Aufnahmedaten, insbesondere der segmentierten Aufnahme, in einem chirurgischen Assistenzsystem; vorzugsweise Handhabung Endoskop und/oder Erstellen einer intrakorporalen Aufnahme; Anvisieren und erfassen anatomischer Landmarken in der nativen endoskopischen intrakorporalen Aufnahme; Bestimmen und Erfassen von korrespondierenden Landmarken in den 3D-Aufnahmedaten; Registrieren der beiden Aufnahmen (der intrakorporalen Aufnahme und den 3D-Aufnahmedaten) über die erfassten Landmarken; (Erfassen und) Bestimmen einer Bewegung des Aufnahmekopfs, insbesondere des Endoskops, insbesondere durch Verwendung von zumindest einem endoskopinternen Sensor und/oder einer Bildanalyse/Aufnahmeanalyse der endoskopischen intrakorporalen Aufnahme; Übertragen der ermittelten Bewegung auf die 3D-Aufnahme; und korrelierte Ausgabe der beiden Aufnahmen, insbesondere nebeneinander und/oder überlagert.

Vorzugsweise weist das Darstellungsverfahren einen weiteren Schritt einer erneuten Registrierung auf.

Gemäß einer weiteren bevorzugten Ausführungsform kann das Darstellungsverfahren die Schritte aufweisen: Detektieren eines medizinischen Instruments und seiner Lage in der intrakorporalen Aufnahme; Lagekorrekte virtuelle Überlagerungsdarstellung / Darstellung des detektierten Instruments in den 3D-Aufnahmedaten;

Vorzugsweise kann das Darstellungsverfahren den Schritt aufweisen: Lagekorrektes Darstellen des virtuellen Endoskops, insbesondere des virtuellen Aufnahmekopfs in den 3D-Aufnahmedaten.

Gemäß einer weiteren bevorzugten Ausführungsform kann das Darstellungsverfahren den Schritt aufweisen: Einblenden eines vorgeplanten medizinischen Instruments, insbesondere eines vorgeplanten Trokars, in der intrakorporalen Aufnahme und/oder den 3D-Aufnahmedaten.

Insbesondere kann das Darstellungsverfahren die Schritte aufweisen: Erfassen einer Eingabe einer Ansichtenauswahl, insbesondere einer Auswahl aus einer Menge einer Vogelperspektive, einer Endoskopperspektive, einer Seitenansicht und einer Bodenansicht; Anwenden der Ansichtsauswahl auf die Ansicht der 3D-Aufnahmedaten, so dass in der Korrelationsdarstellung die ausgewählte Ansicht dargestellt wird.

Vorzugsweise kann das Darstellungsverfahren ferner die Schritte aufweisen: Bestimmen, anhand der erfassten Landmarken und der bestimmten korrespondierenden Landmarken, einer Deformation einer realen anatomischen Struktur, insbesondere einer Deformation gegenüber einer initialen anatomischen Struktur; und Übertragen dieser bestimmten Deformation auf die virtuelle anatomische Struktur in den 3D-Aufnahmedaten, um die 3D-Aufnahmedaten entsprechend anzupassen. Es kann also anhand von korrespondierenden Landmarken und deren relativer Änderung, insbesondere zu initialen Landmarken, eine Deformation bestimmt werden, und diese Deformation dann auf die 3D-Aufnahmedaten entsprechend übertragen werden, um diese zu korrigieren. Vor allem bei einer Registrierung mittels Oberflächen-Registrierung hat dieses Verfahren Vorteile, bei der nun nicht nur eine starre Korrelation mit den 3D-Aufnahmedaten gemacht wird, sondern ein segmentiertes Objekt, wie etwa eine Leber, in den 3D-Aufnahmedaten entsprechend deformiert wird, so dass die 3D-Aufnahmedaten zu der durch das Endoskop aufgenommenen realen 3D-Strukturen passt. Diese Schritte kann insbesondere die Steuereinheit des Assistenzsystems ausführen.

An dieser Stelle wird darauf hingewiesen, dass Merkmale des Darstellungsverfahrens der vorliegenden Offenbarung sich sowohl auf das chirurgische Assistenzsystem der vorliegenden Offenbarung als auch umgekehrt übertragen lassen.

Hinsichtlich eines computerlesbaren Speichermediums werden die Aufgaben und Ziele der vorliegenden Offenbarung dadurch gelöst, dass das computerlesbare Speichermedium Befehle umfasst, die bei einer Ausführung durch einen Computer diesen veranlassen, die Verfahrensschritte des Aufnahmedarstellungsverfahren gemäß der vorliegenden Offenbarung auszuführen.

Hinsichtlich eines gattungsgemäßen Sterilraums wird die Aufgabe der vorliegenden Offenbarung dadurch gelöst, dass der medizinische Sterilraum ein chirurgisches Assistenzsystem gemäß der vorliegenden Offenbarung aufweist.

### Kurzbeschreibung der Figuren

Die vorliegende Offenbarung wird nachfolgend anhand bevorzugter Ausführungsformen mit Hilfe von begleitenden Figuren erläutert. Es zeigen:
Fig. 1 eine schematische Ansicht eines chirurgischen Assistenzsystems einer ersten bevorzugten Ausführungsform,
Fig. 2 eine schematische Teilansicht eines starren Endoskops eines chirurgischen Assistenzsystems einer weiteren bevorzugten Ausführungsform mit zugehöriger intrakorporaler Aufnahme, dessen Bewegung auf ein virtuelles Endoskop mit einer Ansicht der 3D-Aufnahmedaten übertragen werden;
Fig. 3 eine schematische Ansicht einer aufnahmebasierten Registrierung eines chirurgischen Assistenzsystems;
Fig. 4 eine schematische perspektivische Ansicht eines Videoendoskops, das bei einem chirurgischen Assistenzsystem einer bevorzugten Ausführungsform eingesetzt wird;
Fig. 5 eine schematische Ansicht unterschiedlicher Perspektiven für eine Ansicht der 3D-Aufnahmedaten; und
Fig. 6 ein Flussdiagramm eines Aufnahmedarstellungsverfahrens einer bevorzugten Ausführungsform.

Die Figuren sind lediglich schematischer Natur und sollen nur dem Verständnis der Offenbarung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen.

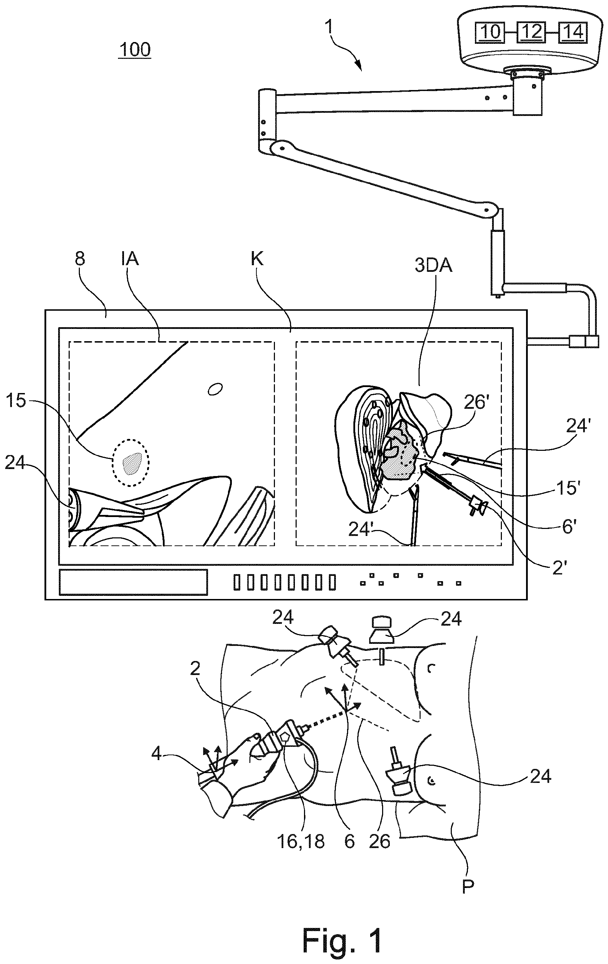

Figur 1 zeigt eine schematische Ansicht eines chirurgisches Assistenzsystems 1 (nachstehend nur Assistenzsystem genannt) einer ersten bevorzugten Ausführungsform zur Verwendung. Das Assistenzsystem 1 kommt in einem medizinischen Sterilraum in Form eines Operationssaals 100 einer bevorzugten Ausführungsform zum Einsatz, um OP-Beteiligte, insbesondere einen Chirurgen, durch geeignete Visualisierung bei einem chirurgischen Eingriff bei einem Patienten P (hier nur schematisch dargestellt) zu unterstützen. In einem mittigen, sterilen, chirurgischen Eingriffsbereich wird an dem Patienten ein minimalinvasiver Eingriff vorgenommen. Ein starres Videoendoskop 2 (nachstehend nur Endoskop genannt) mit einem Handstück / Handgriff 4 und einem beabstandeten frontseitigen bildgebenden Aufnahmekopf 6 steht dafür endoskopschaftseitig in das Körperinnere des Patienten hinein. Der Aufnahmekopf weist für eine Bildgebung eine Endoskop-Videokamera (nicht dargestellt) auf, welche in Richtung einer Längsachse des Endoskops 2 stirnseitig mittels eines CMOS-Sensors eine zweidimensionale intrakorporale Aufnahme IA des Körperinneren des Patienten P als zeitaktuelle (Live-Video) Aufnahme erstellt und digital bereitstellt. Über eine Darstellungsvorrichtung in Form eines OP-Monitors 8 kann dann ein visueller Inhalt für OP-Beteiligte, wie etwa eine Darstellung der intrakorporalen Aufnahme IA wiedergegeben werden. Es wird darauf hingewiesen, dass das Endoskop 2 alternativ auch an seinem Handhabungsabschnitt von einem Roboterarm geführt werden kann.

Ferner weist das chirurgische Assistenzsystem 1 eine Datenbereitstellungseinheit in Form einer Speichereinheit 10 auf in welcher digitale, präoperative 3D-Aufnahmedaten 3DA des zu behandelnden Patienten P digital/computerlesbar hinterlegt sind. Als 3D-Aufnahmedaten 3DA können etwa (segmentierte) MRT-Aufnahmedaten oder CT-Aufnahmedaten hinterlegt sein, die einen Körper des Patienten P oder zumindest einen Teil des Körpers virtuell bzw. digital abbilden.

Zur Erfassung, Verarbeitung und Berechnung sowie zur Steuerung weist das Assistenzsystem 1 eine Steuereinheit 12 auf, die dafür angepasst ist, die intrakorporale Aufnahme IA des Endoskops 2 sowie die 3D-Aufnahmedaten zu verarbeiten sowie den OP-Monitor 8 entsprechend anzusteuern.

Auch weist das chirurgische Assistenzsystem eine Registrierungseinheit 14 auf, die in dieser Ausführungsform als speziell angepasste Steuereinheit 12 ausgebildet ist. Konkret ist die Steuereinheit 12 dafür angepasst, in der intrakorporalen Aufnahme IA des Endoskops 2 anatomische Landmarken 15 zu erfassen sowie korrespondierende anatomische Landmarken 15' in den bereitgestellten 3D-Aufnahmedaten zu bestimmen. In der intrakorporalen Aufnahme IA des Endoskops 2, welches in dem Körper des Patienten P eingeführt ist und eine anvisierte anatomische Struktur als zweidimensionales Bild aufnimmt, wählt der Chirurg in dieser Ausführungsform drei vordefinierte charakteristische (reale) Landmarken 15 aus. Diese drei Landmarken 15 im realen Raum finden sich ebenfalls als drei (virtuelle) Landmarken 15' im virtuellen Raum wieder. Über die insgesamt zwei mal drei korrespondierenden anatomischen Landmarken 15, 15' wird die intrakorporale Aufnahme IA mit den 3D-Aufnahmedaten initial korreliert, also registriert. Auch wird so das Endoskop 2, insbesondere der Aufnahmekopf 6, relativ zu dem Patienten P registriert. Es wird also eine geometrische Relation zwischen dem virtuellen Aufnahmekopf 6' und dem virtuellen Körper des Patienten P (3D-Aufnahmedaten) hergestellt, die der realen geometrischen Relation zwischen dem realen Aufnahmekopf 6 und dem realen Körper des Patienten P entspricht. In dem virtuellen Raum berechnet die Steuereinheit 12 eine entsprechende Transformationsmatrix und überträgt diese auf den realen Raum. Somit ist dann die Lage des Aufnahmekopfs 6 des realen Endoskops 2 relativ zu dem Körper des Patienten P bestimmt. Mit anderen Worten wird also zunächst die (reale) intrakorporale Aufnahme IA mit den (virtuellen bzw. digitalen) 3D-Aufnahmedaten 3DA über zumindest eine charakteristische Landmarke 15, 15', die in beiden Aufnahmen vorkommt initial korreliert, sprich registriert, um die Lage des (realen) Endoskops 2, insbesondere des Aufnahmekopfs 6, gegenüber dem Patienten P zu registrieren bzw. zu bestimmen.

Insbesondere kann die Steuereinheit 12 dafür angepasst sein, unter Kenntnis von optischen Parametern des Aufnahmekopfs 6 oder eines Abstands von dem Aufnahmekopf 6 zu den anatomischen Landmarken eine solche Ansicht der 3D-Aufnahmedaten 3DA zu ermitteln, in denen diese drei Landmarken in den 3D-Aufnahmedaten 3DA einen im Wesentlichen gleichen Abstand zueinander aufweisen, wie in der intrakorporalen Aufnahme IA.

Das chirurgische Assistenzsystem 1 verfügt zudem über ein Trackingsystem 14, welches kontinuierlich eine Bewegung des Endoskops 2 und des Aufnahmekopfs 6 erfasst und der Steuereinheit 12 als Endoskop-Bewegungsdaten bereitstellt. In dieser Ausführungsform kommt eine Kombination eines endoskopinternen Sensors und einer durch die Steuereinheit 12 ausgeführten Bildanalyse zum Einsatz.

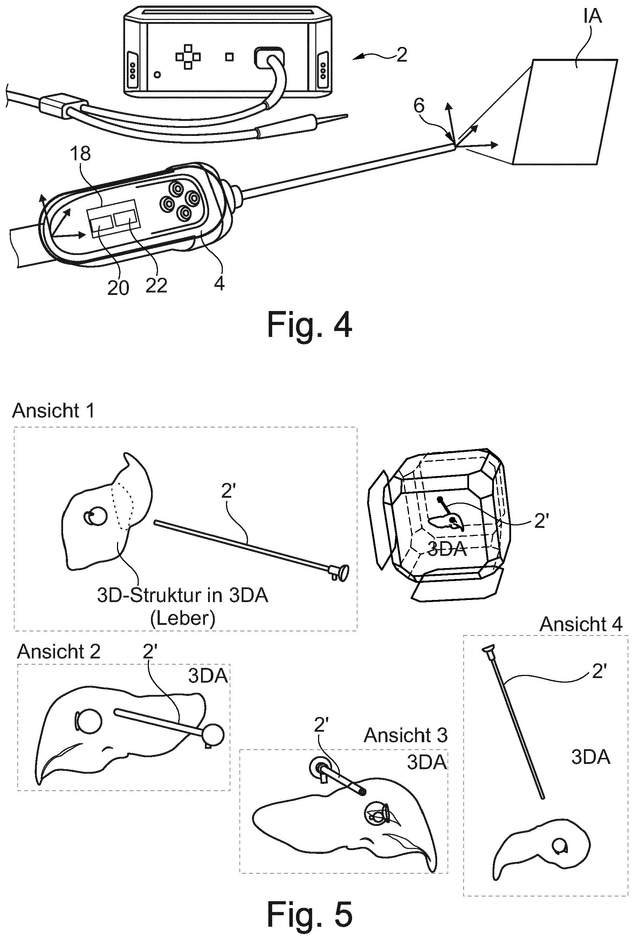

Konkret weist der Handgriff 4 des starren Endoskops 2 eine inertiale Messeinheit (IMU) 18 mit mehreren Inertialsensoren, hier einem Beschleunigungssensor 20 und einem Drehratensensor 21 (Drei-Achsen-Beschleunigungssensor und Drei-Achsen-Gyroskop) für eine Ausbildung als Trägheitsnavigationssystem auf (siehe auch Fig. 4). Die jeweiligen Beschleunigungen in die drei Richtungen ergibt eine Gesamtbeschleunigung in eine Richtung im realen Raum (Vektor). Die erfassten Drehraten um die drei Achsen können beispielsweise als Drehmatrix abgebildet werden. Es wird also kontinuierlich (aufgrund der technischen Beschränkungen angenähert in diskreten Zeitabständen mit möglichst kleinen Zeitintervallen) eine Bewegung des Endoskops im realen Raum erfasst und als Endoskop-Bewegungsdaten mit Translationen und Rotationen an die Steuereinheit 12 weitergegeben. Über die fixe geometrische Relation von Handgriff 4 zu Aufnahmekopf 6 wird mit der IMU 18 nicht nur die Bewegung des Handgriffs 4, sondern auch die des Aufnahmekopfs 6 erfasst.

Zudem ist bei dem chirurgischen Assistenzsystem 1 die Steuereinheit 12 dafür angepasst, eine Bildanalyse anhand der intrakorporalen Aufnahme IA durchzuführen. Die Steuereinheit 12 führt eine Datenverarbeitung dahingehend aus, dass sie eine zeitliche Veränderung der anatomischen Strukturen erfasst und damit eine Bewegung der Strukturen (insbesondere Richtungsänderung) und anhand dieser Veränderungen eine Bewegung des Aufnahmekopfs 6 und damit des Endoskops 2 bestimmt. Damit liegt neben sensorischen Endoskop-Bewegungsdaten auch bildanalytische Endoskop-Bewegungsdaten vor, die miteinander verknüpft werden können, um Messfehler zu minimieren und eine Genauigkeit zu erhöhen. Vorliegend wird ein Mittelwert der beiden Endoskop-Bewegungsdaten gebildet und als Ergebnis der Steuereinheit 12 bereitgestellt.

Da die Steuereinheit 12 ist zusätzlich dafür angepasst, eine Korrelationsdarstellung K mit einer Nebeneinanderdarstellung von sowohl einer Darstellung der intrakorporalen Aufnahme IA als auch einer Darstellung einer Ansicht der 3D-Aufnahmedaten 3DA zu erzeugen, in welcher die intrakorporale Aufnahme IA und die Ansicht der 3D-Aufnahmedaten 3DA hinsichtlich des Endoskops 2, insbesondere des Aufnahmekopfs 6, zueinander korreliert sind. Hierfür werden die erfassten Endoskop-Bewegungsdaten auf zumindest eine virtuelle Position und/oder Orientierung eines virtuellen Aufnahmekopfs 6', insbesondere eines virtuellen Endoskops 2', in der Ansicht der 3D-Aufnahmedaten 3DA für eine korrelierte Bewegung übertragen. Insbesondere wird der virtuelle Aufnahmekopf 4' in gleicher Weise mitbewegt wie der reale Aufnahmekopf 4. Eine Ansicht der 3D-Aufnahmedaten wird entsprechend angepasst. Die Steuereinheit 12 ist schließlich dafür angepasst, die so erzeugte Korrelationsdarstellung K über den OP-Monitor 8 visuell auszugeben. Die Korrelationsdarstellung K ist eine Nebeneinanderdarstellung der endoskopischen intrakorporalen Aufnahme IA und der präoperativen 3D-Aufnahmedaten, wobei beide Aufnahmen in Bezug auf die Bewegung des Endoskops korreliert werden.

Die Ansicht der 3D-Aufnahmedaten ermöglichen eine globale Orientierung, während die endoskopische intrakorporale Aufnahme eine präzise räumliche Orientierung ermöglicht. Das chirurgische Assistenzsystem 1 der vorliegenden Offenbarung ist dafür angepasst beide Modalitäten nebeneinander und in einer korrelierten Weise anzuzeigen. Hierdurch kann der Chirurg die Operation besser durchführen und die 3D-Aufnahmedaten mit insbesondere präoperativen Informationen direkt nutzen, trotz, dass aufgrund der intraoperativen Verformung des Gewebes die endoskopische intrakorporale Aufnahme und die 3D-Aufnahmedaten nicht immer perfekt übereinstimmen. Für den präzisen Eingriff kann er sich weiterhin auf die endoskopische intrakorporale Aufnahme verlassen. Die korrelierten 3D-Aufnahmedaten bzw. 3D-Bilder ermöglichen es dem Chirurgen, kritische Strukturschäden zu vermeiden, sein Operationsziel besser zu finden und einen präoperativen Plan präzise und effizient auszuführen. Durch die globale Führung mit den 3D-Aufnahmedaten kann das Assistenzsystem 1 auch eine Lernkurve bei der Verwendung endoskopischer intrakorporaler Aufnahmen verkürzen. Insbesondere führt eine Ausrichtung einer Position und/oder Orientierung des Endoskops, insbesondere des Aufnahmekopfs, zu den 3D-Aufnahmedaten bzw. zum segmentierten 3D-Modell zu einer verbesserten Orientierung des Chirurgen im interessierenden Bereich.

Nach einer initialen Registrierung kann also bei dem chirurgischen Assistenzsystem 1 der vorliegenden Offenbarung sozusagen die Transformationsmatrix im virtuellen Raum in gleichem Maße verändert werden wie die Transformationsmatrix im realen Raum, um das Endoskop 2', insbesondere den Aufnahmekopf 6`, im virtuellen Raum gleich zu dem Endoskop 2 bzw. dem Aufnahmekopf 6 im realen Raum mitzuführen und die Ansicht der 3D-Aufnahmedaten 3DA entsprechend anzupassen (zu korrelieren), insbesondere mitzubewegen.

Eine initiale Registrierung (als absolute Korrelation) mittels der Registrierungseinheit 14 kann insbesondere wie folgt durchgeführt werden. Es kann dem Aufnahmekopf 6 des Endoskops 2, in dem realen Raum, ein lokales Aufnahmekopf-Koordinatensystem zugewiesen werden. Auch dem Körper des Patienten P kann ein lokales Koordinatensystem zugeordnet werden. Für eine initiale Registrierung muss eine Transformationsmatrix zwischen diesen beiden lokalen Koordinatensystemen berechnet und bereitgestellt werden, um über diese Transformationsmatrix eine initiale Lage (Position und Orientierung) des Aufnahmekopfs und damit insbesondere des Endoskops relativ zu dem Körper des Patienten P zu bestimmen. Auch in dem virtuellen Raum kann ein lokales Aufnahmekopf-Koordinatensystem dem virtuellen Aufnahmekopf 6' sowie ein lokales Koordinatensystem dem "virtuellen Körper" des Patienten zugeordnet werden. Man könnte auch sagen, dass dem realen Körper ein virtueller Körper oder zumindest ein Teilbereich, wie etwa ein Organ, deckungsgleich zugeordnet wird. Damit ist eine Basis-Verknüpfung zwischen dem realen Raum und dem virtuellen Raum hergestellt. Dies ist einleuchtend, da insbesondere präoperative 3D-Aufnahmedaten, wie etwa CT-Aufnahmedaten oder MRT-Aufnahmedaten des gesamten Körpers oder zumindest eines Teilbereichs des Körpers aufgenommen werden, ja den realen Körper 1:1 im Raum abbilden. Die reale intrakorporale Aufnahme IA einer anatomischen Struktur mit der zumindest einer erfassten Landmarke findet sich auch in den virtuellen 3D-Aufnahmedaten wieder und ermöglicht mittels eines Abgleichs eine Registrierung, insbesondere zwischen dem virtuellen Aufnahmekopf 6'und dem virtuellen Körper. Hierdurch wird eine initiale Transformationsmatrix in dem virtuellen Raum gewonnen, die auf den realen Raum "übertragen" werden kann, um eine initiale Registrierung durchzuführen. Folglich ist nach der Registrierung in beiden Räumen zumindest die geometrische Lage des Aufnahmekopfs zu dem Körper des Patienten korreliert und damit auch die intrakorporale Aufnahme zu den 3D-Aufnahmedaten.

Mithilfe des chirurgischen Assistenzsystems 1 der vorliegenden Offenbarung kann also mit einer nur sehr geringen Änderung von Hardware bestehender Endoskope, nämlich durch Ergänzung eines (Standard-)Endoskops mit einem internen (Bewegungs-)Sensor 18 und/oder durch Erfassung der Bewegung des Endoskops 2 über eine externe Kamera wie einer 3D-Stereo-Kamera, sowie durch entsprechende Anpassung einer Steuereinheit, die insbesondere über ein entsprechend hinterlegtes Programm erfolgen kann, ohne zusätzliche platzintensive und fehleranfällige Hardwarevorrichtungen eine intrakorporale Aufnahme IA mit einer Ansicht der 3D-Aufnahmedaten 3DA korreliert und über den OP-Monitor 8 ausgegeben werden. Dieses Assistenzsystem 1 verbessert eine Orientierung eines Chirurgen und damit auch eine Sicherheit eines Patienten P.

Die Steuereinheit 12 ist ferner dafür angepasst mittels einer in der Speichereinheit 10 hinterlegten Datenbank mit geometrisch vordefinierten Strukturen medizinischer Instrumente 24 in der intrakorporalen Aufnahme IA zum einen ein solches medizinisches Instrument 24 zu erkennen, und ferner noch eine zugehörige Lage im realen Raum zu erfassen. Dieses erfasste medizinische Instrument 24 wird dann in den 3D-Aufnahmedaten 3DA in seiner erfassten Lage mittels seiner hinterlegten geometrischen Strukturen virtuell als virtuelles medizinisches Instrument 24' lagekorrekt eingeblendet bzw. virtuell überlagert, so dass das medizinische Instrument 24, 24' in beiden Darstellungen der Aufnahmen IA, 3DA angezeigt wird. So kann eine Orientierung weiter verbessert werden.

Neben der Erkennung von medizinischen Instrumenten 24 im realen Raum ist die Steuereinheit 12 auch dafür angepasst vorgeplante, virtuelle medizinische Instrumente 24', insbesondere einen vorgeplanten Trokar, in der Ansicht der 3D-Aufnahmedaten (3DA) (eingeblendet bzw. überlagert) im virtuellen Raum anzuzeigen. Diese vorgeplanten virtuellen medizinischen Instrumente 24' inklusive ihrer Lage relativ zum (virtuellen) Körper des Patienten sind ebenfalls in der Speichereinheit 10 hinterlegt.

Zudem ist in der Speichereinheit 10 ein geplanter Pfad zum Operationsfeld hinterlegt, der in den 3D-Aufnahmedaten entsprechend eingeblendet werden kann, etwa als Pfeil oder als dreidimensionale Pfadlinie, um den Chirurgen einen Weg zu weisen.

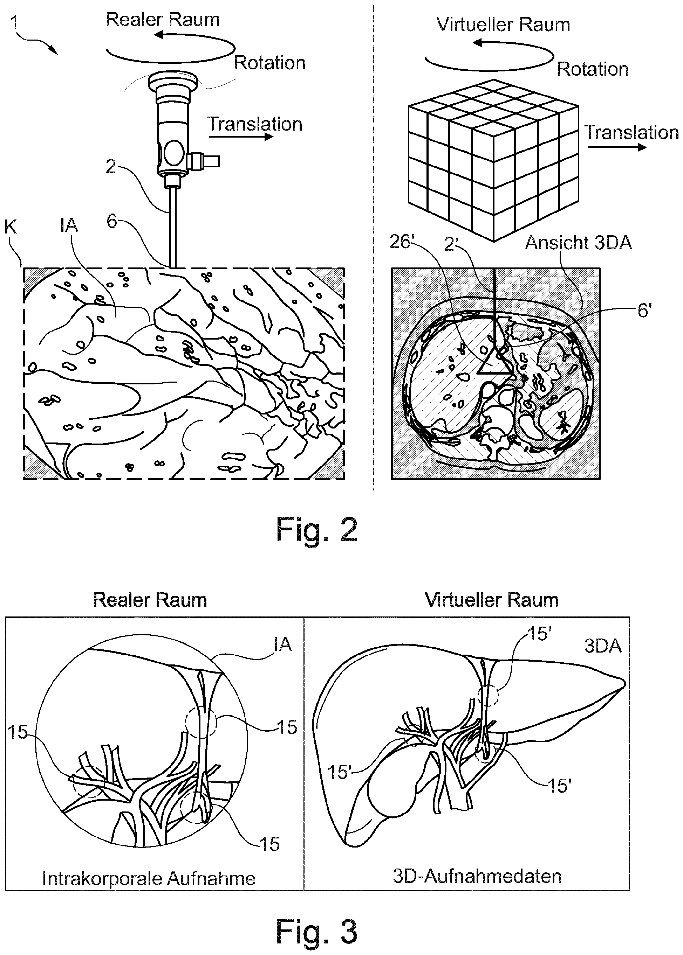

Figur 2 zeigt eine schematische Teilansicht eines Assistenzsystems 1 einer weiteren bevorzugten Ausführungsform mit einer Gegenüberstellung des realen Raums und des virtuellen Raums zur Erläuterung der Funktionsweise. Das Assistenzsystem 1 unterscheidet sich zum Assistenzsystem 1 der Fig.1 in Hauptsache durch die ausgewählte Ansicht der 3D-Aufnahmedaten 3DA. Im linken Teilbereich der Fig. 2 ist der reale Raum und im rechten Teilbereich der virtuelle Raum abgebildet. Das reale Endoskop 2 erfasst stirnseitig über seinen Aufnahmekopf 6 die intrakorporale Aufnahme IA, die dem Chirurgen der lokalen Navigation dient. Das korrespondierende virtuelle Endoskop 2' mit seinem virtuellen Aufnahmekopf 6' wird in einem Segment einer CT-Aufnahme (als 3D-Aufnahmedaten 3DA) an seiner aktuellen Position mit eingeblendetem virtuellem Sichtfeld 26' angezeigt. Für die Korrelationsdarstellung K wird also für die Ansicht der 3D-Aufnahmedaten 3DA ein Segment der CT-Aufnahme verwendet, welches der Position des Aufnahmekopfes 6 in Richtung Superior-Anterior entspricht. In der Ansicht der 3D-Aufnahmedaten wird dann lagekorrekt virtuell der Aufnahmekopf 6' und dessen Orientierung schematisch eingeblendet. Somit sieht der Chirurg auf einen Blick in einfacher und intuitiver Weise in der Ansicht der 3D-Aufnahmedaten 3DA, wo er sich global im Körper des Patienten P befindet.

Wird nun in Fig. 2 nach der Registrierung (wie bereits zu Fig. 1 erläutert), das Endoskop 2 bewegt (siehe Bewegungspfeile realer Raum), so wird diese erfasste Bewegung auf das virtuelle Endoskop 2' mit dem virtuellen Aufnahmekopf 6' übertragen (siehe Bewegungspfeile virtueller Raum). Hierdurch ändert sich einerseits die angezeigte Lage des virtuellen Aufnahmekopfs 6'und zudem wird, wenn sich der Aufnahmekopf 6 in Richtung Superior-Anterior bewegt, ein an dieser Stelle passendes Segment der CT-Aufnahme angezeigt. Auch wird, wenn etwa das Endoskop 2 um seine Längsachse rotiert wird, diese Rotation auf die Ansicht der 3D-Aufnahmedaten 3DA übertragen. Wenn etwa im einfachsten Beispiel das Endoskop 2 genau in Richtung Superior-Anterior ausgerichtet ist und die Ansicht der 3D-Aufnahmedaten 3DA ebenfalls in diese Richtung ausgerichtet ist, so wird die Rotation 1:1 auf die Ansicht übertragen. Wird beispielsweise das Endoskop 2 um 90° im Uhrzeigersinn gedreht, wird auch die Ansicht der 3D-Aufnahmedaten 3DA um 90° im Uhrzeigersinn gedreht. Durch das Assistenzsystem 1 ist also die intrakorporale Aufnahme IA mit der (auswählbaren und veränderbaren) Ansicht der 3D-Aufnahmedaten 3DA korreliert und eine Bewegung des Endoskops 2 wird auf die Ansicht der 3D-Aufnahmedaten 3DA übertragen und die Ansicht entsprechend angepasst.

In der Korrellationsdarstellung können beispielsweise zwei Darstellungen der intrakorporalen Aufnahme enthalten sein. Hier kann eine erste Darstellung eine (Splitscreen-)Livedarstellung im Fluoreszenzmodus sein während die zweite Darstellung das "normale" Endoskopbild zeigt. Es sind also mehrere Systeme und Modi und somit mehrere Livedarstellungen einer intrakorporalen Aufnahme möglich. Gleichfalls können in der Korrelationsdarstellung mehrere Darstellungen von unterschiedlicher Perspektiven derselben 3D-Aufnahmedaten dargestellt werden.

Figur 3 zeigt schematisch eine bevorzugte Ausführungsform des Assistenzsystems 1 mit Registrierung der intrakorporalen Aufnahme IA zu den 3D-Aufnahmedaten 3DA über drei Punkte. In der einfachsten Form wählt der Chirurg in der intrakorporalen Aufnahme IA drei zueinander beabstandet charakteristische Landmarken 15 aus. Diese drei Landmarken 15 finden sich als korrespondierende virtuelle Landmarken 15' in den 3D-Aufnahmedaten 3DA wieder. Entweder kann die Steuereinheit 12 angepasst sein, die Landmarken 15' automatisch zu bestimmen oder der Chirurg kann manuell die drei virtuellen Landmarken 15' in den 3D-Aufnahmedaten bestimmen. Auf diese Weise wird die intrakorporale Aufnahme IA zu der 3D-Aufnahmedaten 3DA als auch das Endoskop 2 zu dem Patienten P registriert.

Figur 4 zeigt in einer detaillierten perspektivischen Teilansicht ein starres Endoskop 2 (mit starrem Schaft) gemäß einer Ausführungsform, welches in der Assistenzvorrichtung 1 der Fign. 1 bis 3 eingesetzt werden kann. Das Endoskop 2 weist den Handgriff 4 und den distalen Aufnahmekopf 6 auf, der in einer Aufnahmerichtung die intrakorporale Aufnahme IA (hier nur schematische dargestellt) optisch erfasst und digital der Steuereinheit 12 bereitstellt. In dem Handgriff 4 ist der endoskopinterne Bewegungssensor in Form einer inertialen Messeinheit 18 für eine Ausbildung eines Trägheitsnavigationssystems vorgesehen, der die Bewegung des Endoskops 2 erfasst. Über definierte geometrische Parameter zwischen der Position des internen Sensors 18 und der Spitze des Endoskops 2 mit dem Aufnahmekopf 6, kann auch eine Bewegung des Aufnahmekopfs 6 durch die Steuereinheit 12 berechnet werden. Diese Bewegung wird dann auf die 3D-Aufnahmedaten 3DA in dem virtuellen Raum entsprechend übertragen. Auch sind in Fig. 4 drei lokale Koordinatensysteme gezeigt. Ein erstes lokales Koordinatensystem ist das des Handstücks, ein zweites lokales Koordinatensystem ist das des Aufnahmekopfs und ein drittes lokales Koordinatensystem ist das Bild-Koordinatensystem der Intrakorporalen Aufnahme. Alle drei Koordinatensystem lassen sich über eine geeignete (serielle) Transformation miteinander verknüpfen, so dass (insbesondere mit entsprechenden (geometrischen) Daten zum Endoskop) von einem Koordinatensystem auf ein anderes Koordinatensystem geschlossen werden kann.

Figur 5 zeigt beispielhafte unterschiedliche Ansichten der 3D-Aufnahmedaten, die mittels einer Ansichtenauswahleinheit des Assistenzsystems 1 aus Fig. 1 durch einen Chirurgen auswählbar sind. Diese Darstellung der Ansicht der 3D-Aufnahmedaten 3DA wird dann in der Korrelationsdarstellung K wiedergegeben, um dem Chirurgen eine flexible Anpassung und verbesserte Orientierung zu bieten. So kann der Chirurg etwa analysieren, ob ein Versatz zwischen der Längsachse des starren Endoskops 2 und der Ziel-Operationsfeld besteht, wenn er eine Seitenansicht, eine Vorderansicht und eine Draufansicht auswählen kann, deren Ansichtsrichtungen jeweils senkrecht zueinander stehen.

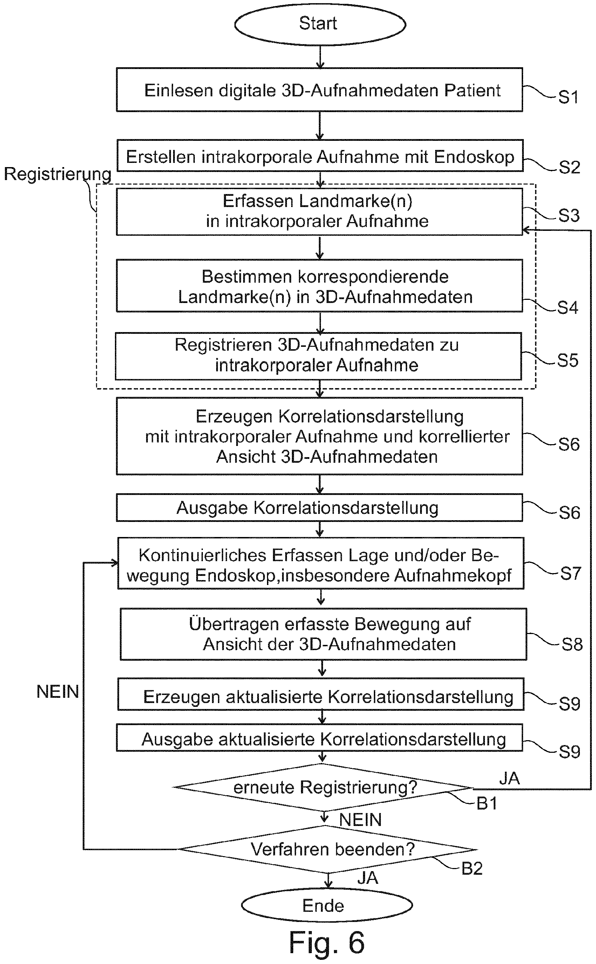

Figur 6 zeigt ein Flussdiagramm eines Aufnahmedarstellungsverfahren zur zentralen korrelierten Darstellung von zwei unterschiedlichen Aufnahmen, insbesondere bei einem chirurgischen Assistenzsystem 1 gemäß einer bevorzugten Ausführungsform der vorliegenden Offenbarung. Es wird an dieser Stelle darauf hingewiesen, dass die Reihenfolge der Schritte auch veränderbar ist.

In einem ersten Schritt S1 werden digitale 3D-Aufnahmedaten 3DA, insbesondere präoperative 3D-Aufnahmedaten, eines Patienten P, eingelesen. Beispielsweise können die eingelesenen 3D-Aufnahmedaten wie einer CT-Aufnahme des gesamten Körpers des Patienten P, in einer Speichereinheit mit entsprechender Segmentierung hinterlegt werden.

In einem nächsten Schritt S2 wird eine intrakorporale Aufnahme IA durch das Endoskop 2, hier durch den Aufnahmekopf, erstellt oder erfasst. Es sei angemerkt, dass die Reihenfolge der Schritte S1 und S2 unerheblich ist, so dass auch zuerst die intrakorporale Aufnahme IA und dann das Einlesen erfolgen kann oder beide Schritte S1 und S2 gleichzeitig erfolgen können.

In einem darauffolgenden Schritt S3 werden in der intrakorporalen Aufnahme IA Landmarken 15 anvisiert und erfasst, insbesondere drei Landmarken 15. Nach Erfassen der Landmarken 15 werden im Schritt S4 korrespondierende Landmarken 15' in den 3D-Aufnahmedaten 3DA bestimmt. Über die erfassten Landmarken 15, 15' werden in einem Schritt S5 die 3D-Aufnahmedaten 3DA zu der intrakorporalen Aufnahme IA registriert. Die Schritte S3 bis S5 bilden damit zusammen den Schritt der Registrierung.

In einem Schritt S6 wird eine Korrelationsdarstellung K mit einer Darstellung der intrakorporalen Aufnahme IA und einer korrelierten Darstellung einer Ansicht der 3D-Aufnahmedaten 3DA erzeugt und ausgegeben, etwa durch die Darstellungsvorrichtung in Form des OP-Monitors 8. Man kann auch sagen, dass eine Transformations-Korrelation der endoskopischen intrakorporalen Aufnahme (endoskopisches Bild) mit den präoperativen 3D-Aufnahmedaten (3D-Bild) durchgeführt wird.

In einem Schritt S7 wird (kontinuierlich) eine Lage und/oder eine Bewegung des Endoskops 2, insbesondere über den Handhabungsabschnitt 4 indirekt auch des Aufnahmekopfs 6, erfasst. Diese erfasste Bewegung kann als Endoskop-Bewegungsdaten bereitgestellt werden.

In einem Schritt S8 wird die erfasste Bewegung des Endoskops 2 mit dem Aufnahmekopf 6, auf eine virtuelle Position und Orientierung eines virtuellen Aufnahmekopfs 6' eines virtuellen Endoskops 2', in der Ansicht der 3D-Aufnahmedaten 3DA übertragen.

In einem darauffolgenden Schritt S9 wird eine aktualisierte Korrelationsdarstellung K mit einer Nebeneinanderdarstellung der intrakorporalen Aufnahme IA und der aktualisierten Ansicht der 3D-Aufnahmedaten 3DA erzeugt, welche durch etwa den OP-Monitor 8 angezeigt wird. Auf diese Weise wird dem Chirurgen eine zeitaktuelle, korrelierte Darstellung der intrakorporalen Aufnahme IA und der Ansicht der 3D-Aufnahmedaten 3DA bereitgestellt.

In einer ersten Bedingung B1 wird hiernach geprüft, ob eine Neuregistrierung durchgeführt werden soll. Falls eine erneute Registrierung durchgeführt werden soll, so springt das Darstellungsverfahren zu Schritt S3 der Erfassung der Landmarken 15.

Falls keine erneute Registrierung durchgeführt werden soll, so springt das Verfahren zu einer zweiten Bedingung B2 und prüft, ob das Verfahren beendet werden soll. Falls das Verfahren nicht beendet werden soll, so springt das Verfahren wieder zum Schritt S7 der Bewegungserfassung und es wird eine erneute Iteration ausgeführt.

Falls die Bedingung B2 ergibt, dass das Verfahren beendet werden soll, so wird die Schleife unterbrochen und das Aufnahmedarstellungsverfahren final beendet.

Bei einer erneuten Registrierung müssen die Landmarken nicht zwangsläufig neu definiert werden. Es können insbesondere auch die zuvor bestimmten Landmarken verwendet werden, die nur "neu vermessen" bzw. korreliert werden.

Insbesondere ist das vorstehende Aufnahmedarstellungsverfahren in Form von Befehlen auf einem computerlesbaren Speichermedium abgespeichert und, wenn es durch einen Computer gelesen und ausgeführt wird, veranlasst den Computer die Verfahrensschritte auszuführen.

### Bezugszeichenliste

- 1: chirurgisches Assistenzsystem
- 2: Endoskop
- 2': virtuelles Endoskop
- 4: Handgriff
- 6: Aufnahmekopf
- 6`: virtueller Aufnahmekopf
- 8: OP-Monitor
- 10: Speichereinheit
- 12: Steuereinheit
- 14: Registrierungseinheit
- 15: Landmarke
- 15': virtuelle Landmarke
- 16: Trackingsystem
- 18: IMU
- 20: Beschleunigungssensor
- 22: Drehratensensor
- 24: Medizinisches Instrument
- 24': Virtuelles medizinisches Instrument
- 26: Sichtfeld Endoskop
- 26': Virtuelles Sichtfeld Endoskop
- 100: Operationssaal

- S1: Schritt Einlesen Aufnahmedaten
- S2: Schritt Erstellen oder Erfassen einer intrakorporalen Aufnahme
- S3: Schritt Erfassen zumindest einer Landmarke in intrakorporaler Aufnahme
- S4: Schritt Bestimmen korrespondierende Landmarken in 3D-Aufnahmedaten
- S5: Schritt Registrieren über korrespondierende Landmarken
- S6: Schritt Erzeugen Korrelationsdarstellung
- S7: Schritt Erfassen Bewegung Endoskop
- S8: Schritt Korrelieren durch Übertragen der erfassten Bewegung
- S9: Schritt Erzeugen und Ausgeben aktualisierte Korrelationsdarstellung
- B1: Bedingung Neuregisterierung?
- B2: Bedingung Verfahren beenden?

- P: Patient
- IA: Intrakorporale Aufnahme
- 3DA: 3D-Aufnahmedaten
- K: Korrelationsdarstellung

## Patentansprüche

1. Chirurgisches Assistenzsystem (1) zur Verwendung bei einem chirurgischen Eingriff bei einem Patienten (P), aufweisend:
eine Darstellungsvorrichtung, insbesondere einen OP-Monitor (8), zur Darstellung eines visuellen Inhalts, insbesondere eines Bildes,
ein Endoskop (2), insbesondere ein Videoendoskop, mit einem, vorzugsweise distalen, bildgebenden Aufnahmekopf (6), welches dafür vorgesehen und angepasst ist, eine intrakorporale Aufnahme (IA) des Patienten (P) zu erstellen,
eine Datenbereitstellungseinheit, insbesondere eine Speichereinheit (10), welche dafür angepasst ist, digitale 3D-Aufnahmedaten (3DA), insbesondere präoperative 3D-Aufnahmedaten (3DA), des Patienten (P) bereitzustellen, insbesondere aufzubereiten und bereitzustellen,
eine Steuereinheit (12), die dafür angepasst ist, die intrakorporale Aufnahme (IA) des Endoskops (2) sowie die 3D-Aufnahmedaten (3DA) zu verarbeiten,
**gekennzeichnet durch**
eine Registrierungseinheit (14), die dafür angepasst ist, in der intrakorporalen Aufnahme (IA) zumindest eine, insbesondere vorbestimmte, anatomischen Landmarke (15) und/oder anatomische Orientierung zu erfassen sowie eine korrespondierende anatomische Landmarke (15') und/oder Orientierung in den bereitgestellten 3D-Aufnahmedaten (3DA) zu bestimmen und über die korrespondierenden anatomischen Landmarken (15, 15') die intrakorporale Aufnahme (IA) mit den 3D-Aufnahmedaten (3DA), zumindest initial, zu registrieren und das Endoskop (2), insbesondere den Aufnahmekopf (6), relativ zu dem Patienten (P) zu registrieren,
ein Trackingsystem (16), welches dafür angepasst ist, kontinuierlich eine Lage und/oder eine Orientierung und/oder eine Bewegung des Endoskops (2), insbesondere des Aufnahmekopfs (6), zu erfassen und der Steuereinheit (12) als Endoskop-Bewegungsdaten bereitzustellen,
wobei die Steuereinheit (12) ferner dafür angepasst ist, eine Korrelationsdarstellung (K) mit sowohl mindestens einer Darstellung der intrakorporalen Aufnahme (IA) als auch mindestens einer Darstellung einer Ansicht der 3D-Aufnahmedaten (3DA) zu erzeugen, in welcher die intrakorporale Aufnahme (IA) und die Ansicht der 3D-Aufnahmedaten (3DA) hinsichtlich des Endoskops (2), insbesondere des Aufnahmekopfs (6), zueinander korreliert sind, indem die erfassten Endoskop-Bewegungsdaten auf zumindest eine virtuelle Position und/oder Orientierung eines virtuellen Aufnahmekopfs (6'), insbesondere eines virtuellen Endoskops (2'), in der Ansicht der 3D-Aufnahmedaten (3DA) für eine korrelierte Bewegung übertragen werden, und die Steuereinheit (12) dafür angepasst ist die so erzeugte Korrelationsdarstellung (K) durch die Darstellungsvorrichtung visuell auszugeben, wobei:
das Trackingsystem (16) als ein endoskopinternes Trackingsystem (16) ausgebildet ist und das Endoskop (2), insbesondere ein Handhabungsabschnitt (4) und/oder der Aufnahmekopf (6), einen endoskopinternen Bewegungssensor, insbesondere einen Beschleunigungssensor (20) und/oder einen Drehratensensor (22), besonders bevorzugt eine inertiale Messeinheit (18), zur Erfassung der Endoskop-Bewegungsdaten aufweist, und/oder
für das Trackingsystem (16) die Steuereinheit (12) dafür angepasst ist, über eine Bildanalyse von sich bewegenden anatomischen Strukturen in der intrakorporalen Aufnahme (IA) die Bewegung des Endoskops, insbesondere des Aufnahmekopfs, zu bestimmen.

2. Chirurgisches Assistenzsystem (1) nach Anspruch 1, **dadurch gekennzeichnet, dass** das Trackingsystem (16) den in dem Endoskop vorgesehenen Beschleunigungssensor (20) und/oder Drehratensensor (22), insbesondere die inertiale Messeinheit (18), aufweist, und zudem die Steuereinheit (12) dafür angepasst ist, über die Bildanalyse die Endoskop-Bewegungsdaten zu erfassen, wobei aus den zwei Ergebnissen der Endoskop-Bewegungsdaten ein kombinierter Wert der beiden Ergebnisse, insbesondere ein angepasster Mittelwert oder ein gewichteter Wert, als Endoskop-Bewegungsdaten berechnet wird.

3. Chirurgisches Assistenzsystem (1) nach einem der vorstehenden Ansprüche, **dadurch gekennzeichnet, dass** die Steuereinheit (12) dafür angepasst ist, insbesondere auf Basis einer in der Speichereinheit (10) hinterlegten Datenbank mit geometrisch vordefinierten Strukturen medizinischer Instrumente (24'), in der intrakorporalen Aufnahme (IA) zumindest ein medizinisches Instrument (24) lagekorrekt zu erkennen und vorzugsweise das erkannte medizinische Instrument (24) in der Ansicht der 3D-Aufnahmedaten (3DA) lagekorrekt anzuzeigen, so dass das medizinische Instrument (24, 24') in beiden Aufnahmen (IA, 3DA) angezeigt wird.

4. Chirurgisches Assistenzsystem (1) nach einem der vorstehenden Ansprüche, **dadurch gekennzeichnet, dass** die Registrierungseinheit (14) über die initiale Registrierung hinaus zu zumindest einem weiteren Zeitpunkt eine erneute Registrierung durchführt, um eine Genauigkeit der Korrelation weiter zu erhöhen.

5. Chirurgisches Assistenzsystem (1) nach einem der vorstehenden Ansprüche, **dadurch gekennzeichnet, dass** das Endoskop (2), insbesondere ein Handhabungsabschnitt des Endoskops (2), robotergeführt ist, insbesondere mittels eines Roboterarms, und das Trackingsystem (16) die Lage und/oder Orientierung und/oder Bewegung des Endoskops (2), insbesondere des Aufnahmekopfs (6), über eine zugehörige Lage und/oder Orientierung und/oder Bewegung des Roboters, insbesondere des Roboterarms, und damit über den Roboter bestimmt.

6. Chirurgisches Assistenzsystem (1) nach einem der vorstehenden Ansprüche, **dadurch gekennzeichnet, dass** die Steuereinheit (12) dafür angepasst ist, in der Korrelationsdarstellung (K) in der Darstellung der Ansicht der 3D-Aufnahmedaten (3DA) und/oder der intrakorporalen Aufnahme (IA) zusätzlich zu dem Endoskop (2; 2'), in der Speichereinheit (10) hinterlegte vorgeplante medizinische Instrumente (24') und/oder Implantate, insbesondere einen vorgeplanten Trokar, lagekorrekt anzuzeigen.

7. Chirurgisches Assistenzsystem (1) nach einem der vorstehenden Ansprüche, **dadurch gekennzeichnet, dass** die Steuereinheit (12) dafür angepasst ist, in der Korrelationsdarstellung (K) in der Darstellung der Ansicht der 3D-Aufnahmedaten (3DA) und/oder der intrakorporalen Aufnahme (IA) einen geplanten Pfad und/oder Annotationen anzuzeigen, um einem Chirurgen einen Weg zum Operationsfeld zu weisen.

8. Chirurgisches Assistenzsystem (1) nach einem der vorstehenden Ansprüche, **dadurch gekennzeichnet, dass** die Steuereinheit (12) dafür angepasst ist, die Ansicht der 3D-Aufnahmedaten (3DA)
als 3D-Szene oder
als zweidimensionale Querschnitte relativ zu einem Bild-Koordinatensystem des Endoskops (2) und/oder entlang einer Aufnahmeachse des Endoskops (2), insbesondere des Aufnahmekopfs (6), oder
als virtuelles Endoskopbild zu erstellen.

9. Computerlesbares Speichermedium, umfassend Befehle, die bei einer Ausführung durch einen Computer diesen veranlassen, die Verfahrensschritte eines Aufnahmedarstellungsverfahrens zur zentralen, korrelierten Darstellung von zwei unterschiedlichen Aufnahmen, insbesondere bei einem chirurgischen Assistenzsystem (1) gemäß einem der Ansprüche 1 bis 8, auszuführen, das **gekennzeichnet ist durch** die Schritte:
(S1) Einlesen von digitalen 3D-Aufnahmedaten (3DA), insbesondere präoperativen 3D-Aufnahmedaten (3DA), eines Patienten (P);
(S2) Erstellen oder Erfassen einer intrakorporalen Aufnahme (IA) durch das Endoskop (2) nach Anspruch 1-8;
(S3) Erfassen von zumindest einer Landmarke (15) und/oder anatomischen Orientierung in der intrakorporalen Aufnahme (IA);
(S4) Bestimmen, durch eine Registrierungseinheit (14), einer korrespondierenden Landmarke (15') und/oder Orientierung in den 3D-Aufnahmedaten (3DA);
(S5) Registrieren, durch die Registrierungseinheit (14), der 3D-Aufnahmedaten (3DA) zu der intrakorporalen Aufnahme (IA) über die zumindest eine erfasste korrespondierende Landmarke (15, 15') und/oder Orientierung;
(S6) Erzeugen, durch eine Steuereinheit (12), einer Korrelationsdarstellung (K) mit mindestens einer Darstellung der intrakorporalen Aufnahme (IA) und mindestens einer Darstellung einer Ansicht der 3D-Aufnahmedaten (3DA) sowie vorzugsweise Ausgeben der Korrelationsdarstellung (K) durch die Darstellungsvorrichtung;
(S7) Kontinuierliches Erfassen, durch das Trackingsystem (16) nach Anspruch 1-8, einer Lage und/oder Orientierung und/oder einer Bewegung des Endoskops (2) nach Anspruch 1-8, insbesondere eines Aufnahmekopfs (6);
(S8) Übertragen, durch die Steuereinheit (12), der erfassten Bewegung des Endoskops (2), insbesondere des Aufnahmekopfs (6), auf zumindest eine virtuelle Position und/oder Orientierung eines virtuellen Aufnahmekopfs (6'), insbesondere eines virtuellen Endoskops (2'), in der Ansicht der 3D-Aufnahmedaten (3DA) und
(S9) Erzeugung, durch die Steuereinheit (12), mindestens einer aktualisierten Korrelationsdarstellung (K) mit der intrakorporalen Aufnahme (IA) und der aktualisierten Ansicht der 3D-Aufnahmedaten (3DA) und Ausgeben der Korrelationsdarstellung (K) durch die Darstellungsvorrichtung.

10. Computerlesbares Speichermedium nach Anspruch 9, **dadurch gekennzeichnet, dass** das Aufnahmedarstellungsverfahren ferner die Schritte aufweist:
Bestimmen, anhand der erfassten Landmarken und der bestimmten korrespondierenden Landmarken, insbesondere anhand eines Oberflächenabgleichs, einer Deformation einer realen anatomischen Struktur, insbesondere gegenüber einer initialen anatomischen Struktur mit initialen Landmarken, und
Übertragen dieser bestimmten Deformation auf die virtuelle anatomische Struktur in den 3D-Aufnahmedaten, um die 3D-Aufnahmedaten entsprechend anzupassen und zu korrigieren.

11. Medizinischer Sterilraum, wie etwa ein Operationssaal (100), **dadurch gekennzeichnet, dass** der medizinische Sterilraum ein chirurgisches Assistenzsystem (1) nach einem der Ansprüche 1 bis 8 aufweist.

## Claims

1. A surgical assistance system (1) for use in a surgical intervention on a patient (P), comprising:
a display device, in particular an OR monitor (8), for displaying a visual content, in particular a picture,
an endoscope (2), in particular a video endoscope, with a, preferably distal, imaging recording head (6), which is provided and adapted to create an intracorporeal image (IA) of the patient (P),
a data-providing unit, in particular a storage unit (10), which is adapted to provide, in particular to prepare and provide, digital 3D image data (3DA), in particular preoperative 3D image data (3DA), of the patient (P),
a control unit (12) adapted to process the intracorporeal image (IA) of the endoscope (2) and the 3D image data (3DA),
**characterized by**
a registration unit (14) which is adapted to detect, in the intracorporeal image (IA), at least one, in particular predetermined, anatomical landmark (15) and/or anatomical orientation and to determine a corresponding anatomical landmark (15') and/or orientation in the provided 3D image data (3DA), and to register the intracorporeal image (IA) with the 3D image data (3DA), at least initially, and to register the endoscope (2), in particular the recording head (6), relative to the patient (P),
a tracking system (16) which is adapted to continuously detect a pose and/or an orientation and/or a movement of the endoscope (2), in particular of the recording head (6), and to provide the control unit (12) with endoscope movement data,
wherein the control unit (12) is further adapted to generate a correlation display (K) with both at least a display of the intracorporeal image (IA) and at least a display of a view of the 3D image data (3DA), in which the intracorporeal image (IA) and the view of the 3D image data (3DA) are correlated with respect to the endoscope (2), in particular to the recording head (6), by transferring the detected endoscope movement data to at least one virtual position and/or orientation of a virtual recording head (6'), in particular a virtual endoscope (2'), in the view of the 3D image data (3DA) for a correlated movement, and the control unit (12) is adapted to visually output the correlation display (K) thus generated via the display device, wherein the tracking system (16) is configured as an endoscope-internal tracking system (16) and the endoscope (2), in particular a handling portion (4) and/or the recording head (6), has an endoscope-internal movement sensor, in particular an acceleration sensor (20) and/or a rotation rate sensor (22), particularly preferably an inertial measurement unit (18), for detecting the endoscope movement data, and/or for the tracking system (16), the control unit (12) is adapted to determine the movement of the endoscope, in particular of the recording head, via an image analysis of moving anatomical structures in the intracorporeal image (IA).

2. The surgical assistance system (1) according to claim 1, **characterized in that** the tracking system (16) comprises the acceleration sensor (20) and/or rotation rate sensor (22), in particular the inertial measurement unit (18), provided in the endoscope and furthermore the control unit (12) is adapted to detect the endoscope movement data via the image analysis, wherein a combined value of the two results, in particular an adjusted mean value or a weighted value, is calculated from the two results of the endoscope movement data as endoscope movement data.

3. The surgical assistance system (1) according to one of the preceding claims, **characterized in that** the control unit (12) is adapted, in particular on the basis of a database stored in the storage unit (10) with geometrically predefined structures of medical instruments (24'), to recognize at least one medical instrument (24) in the intracorporeal image (IA) in a correct pose and preferably to display the recognized medical instrument (24) in the view of the 3D image data (3DA) in a correct pose, so that the medical instrument (24, 24') is displayed in both images (IA, 3DA).

4. The surgical assistance system (1) according to one of the preceding claims, **characterized in that** the registration unit (14) performs a re-registration beyond the initial registration at at least one further point in time in order to further increase an accuracy of the correlation.

5. The surgical assistance system (1) according to one of the preceding claims, **characterized in that** the endoscope (2), in particular a handling portion of the endoscope (2), is robot-guided, in particular via a robot arm, and the tracking system (16) determines the pose and/or orientation and/or movement of the endoscope (2), in particular of the recording head (6), via an associated pose and/or orientation and/or movement of the robot, in particular of the robot arm, and thus via the robot.

6. The surgical assistance system (1) according to one of the preceding claims, **characterized in that** the control unit (12) is adapted to display in the correlation display (K) in the display of the view of the 3D image data (3DA) and/or of the intracorporeal image (IA), in addition to the endoscope (2; 2'), pre-planned medical instruments (24') and/or implants, in particular a pre-planned trocar, stored in the storage unit (10) in a correct pose.

7. The surgical assistance system (1) according to one of the preceding claims, **characterized in that** the control unit (12) is adapted to display a planned path and/or annotations in the correlation display (K) in the display of the view of the 3D image data (3DA) and/or the intracorporeal image (IA) in order to guide a surgeon to the surgical field.

8. The surgical assistance system (1) according to one of the preceding claims, **characterized in that** the control unit (12) is adapted to generate the view of the 3D image data (3DA)
as 3D scene or
as two-dimensional cross-sections relative to a picture-coordinate system of the endoscope (2) and/or along an image axis of the endoscope (2), in particular of the recording head (6), or
as a virtual endoscope image.

9. A computer-readable storage medium comprising instructions which, when executed by a computer, cause the computer to perform the method steps of an image display method for the central, correlated display of two different images, in particular in a surgical assistance system (1) according to one of claims 1 to 8, **characterized by the** steps:
(S1) reading in digital 3D image data (3DA), in particular pre-operative 3D image data (3DA), of a patient (P);
(S2) creating or detecting an intracorporeal image (IA) through an endoscope (2);
(S3) detecting at least one landmark (15) and/or anatomical orientation in the intracorporeal image (IA);
(S4) determining, by a registration unit (14), a corresponding landmark (15') and/or orientation in the 3D image data (3DA);
(S5) registering, by a registration unit (14), the 3D image data (3DA) to the intracorporeal image (IA) via the at least one detected corresponding landmark (15, 15') and/or orientation;
(S6) generating, by a control unit (12), a correlation display (K) with at least one display of the intracorporeal image (IA) and at least one display of a view of the 3D image data (3DA) and preferably outputting the correlation display (K) by the display device;
(S7) continuously detecting, by the tracking system (16) according to claims 1-8, a pose and/or orientation and/or a movement of the endoscope (2) according to claims 1-8, in particular of a recording head (6);
(S8) transferring, by the control unit (12), the detected movement of the endoscope (2), in particular of the recording head (6), to at least one virtual position and/or orientation of a virtual recording head (6'), in particular of a virtual endoscope (2'), in the view of the 3D image data (3DA), and
(S9) generating, by the control unit (12), at least one updated correlation display (K) with the intracorporeal image (IA) and the updated view of the 3D image data (3DA) and outputting the correlation display (K) by the display device.

10. The computer-readable storage medium according to claim 9, **characterized in that** image display method furthermore comprises the steps of:
determining, based on the detected landmarks and the determined corresponding landmarks, in particular on the basis of a surface matching, a deformation of a real anatomical structure, in particular relative to an initial anatomical structure with initial landmarks, and
transferring this determined deformation to the virtual anatomical structure in the 3D image data in order to adjust and correct the 3D image data accordingly.

11. A medical sterile space, such as an operation room (100), **characterized in that** the medical sterile space comprises a surgical assistance system (1) according to one of claims 1 to 8.

## Revendications

1. Système d'assistance chirurgicale (1) pour utilisation lors d'une intervention chirurgicale auprès d'un patient (P), présentant :
un dispositif de représentation, en particulier un moniteur OP (8) pour la représentation d'un contenu visuel, en particulier d'une image,
un endoscope (2), en particulier un endoscope vidéo, avec une tête d'enregistrement (6) de formation d'image, de préférence distale, qui est prévue et adaptée afin d'établir un enregistrement intracorporel (IA) du patient (P),
une unité de fourniture de données, en particulier une unité de mémoire (10) qui est adaptée afin de fournir, en particulier de préparer et de fournir des données d'enregistrement en 3D numériques (3DA), en particulier des données d'enregistrement en 3D pré-opérationnelles, du patient (P),
un dispositif de commande (12) qui est adapté afin de traiter l'enregistrement intracorporel (IA) de l'endoscope (2) ainsi que les données d'enregistrement en 3D (3DA),
**caractérisé par**
une unité d'enregistrement (14) qui est adaptée afin de détecter dans l'enregistrement intracorporel (IA) au moins un point de repère (15) anatomique, en particulier prédéterminé et/ou une orientation anatomique ainsi que de déterminer un point de repère (15') anatomique correspondant et/ou une orientation dans les données d'enregistrement en 3D (3DA) fournies et par le biais des points de repère (15, 15') anatomiques correspondants d'enregistrer l'enregistrement intracorporel (IA) avec les données d'enregistrement en 3D (3DA), au moins initialement, et d'enregistrer l'endoscope (2), en particulier la tête d'enregistrement (6), par rapport au patient (P),
un système de suivi (16) qui est adapté afin de détecter en continu une position et/ou une orientation et/ou un déplacement de l'endoscope (2), en particulier de la tête d'enregistrement (6) et de la/le fournir au dispositif de commande (12) comme données de déplacement de l'endoscope,
dans lequel le dispositif de commande (12) est de plus adapté afin de générer une représentation de corrélation (K) avec non seulement au moins une représentation de l'enregistrement intracorporel (IA) mais aussi au moins une représentation d'une vue des données d'enregistrement en 3D, dans laquelle l'enregistrement intracorporel (IA) et la vue des données d'enregistrement en 3D (3DA) sont corrélés l'un par rapport à l'autre en ce qui concerne l'endoscope, en particulier la tête d'enregistrement (6), en ce que les données de déplacement de l'endoscope détectées sont transmises sur au moins une position et/ou orientation virtuelle d'une tête d'enregistrement (6') virtuelle, en particulier d'un endoscope (2') virtuel, dans la vue des données d'enregistrement en 3D (3DA) pour un déplacement corrélé, et le dispositif de commande (12) est adapté afin de sortir visuellement la représentation de corrélation (K) ainsi généré par le dispositif de représentation, dans lequel :
le système de suivi (16) est réalisé comme un système de suivi (16) interne à l'endoscope et l'endoscope (2), en particulier une section de manipulation (4) et/ou la tête d'enregistrement (6), présente un capteur de mouvement interne à l'endoscope, en particulier un capteur d'accélération (20) et/ou un capteur de vitesse de rotation (22), le plus préférentiellement une unité de mesure inertielle (18) pour la détection des données de mouvement de l'endoscope, et/ou
pour le système de suivi (16), le dispositif de commande (12) est adapté afin de déterminer le déplacement de l'endoscope, en particulier de la tête d'enregistrement par le biais d'une analyse d'image de structures anatomiques se déplaçant dans l'enregistrement intracorporel (IA).

2. Système d'assistance chirurgicale (1) selon la revendication 1, **caractérisé en ce que** le système de suivi (16) présente le capteur d'accélération (20) prévu dans l'endoscope et/ou capteur de vitesse de rotation (22), en particulier l'unité de mesure inertielle (18), et en outre le dispositif de commande (12) est adapté afin de détecter les données de déplacement de l'endoscope par le biais de l'analyse d'image, dans lequel une valeur combinée des deux résultats, en particulier une valeur moyenne adaptée ou une valeur pondérée est calculée comme données de déplacement de l'endoscope à partir des deux résultats des données de déplacement de l'endoscope.

3. Système d'assistance chirurgicale (1) selon l'une quelconque des revendications précédentes, **caractérisé en ce que** le dispositif de commande (12) est adapté afin de détecter en position correcte des instruments médicaux (24'), en particulier sur la base d'une base de données enregistrée dans l'unité de mémoire (10) avec des structures prédéfinies géométriquement, dans l'enregistrement intracorporel (IA) au moins un instrument médical (24) et de préférence d'afficher en position correcte l'instrument médical détecté (24) dans la vue des données d'enregistrement en 3D (3DA), de sorte que l'instrument médical (24, 24') soit affiché dans les deux enregistrements (IA, 3DA).

4. Système d'assistance chirurgicale (1) selon l'une quelconque des revendications précédentes, **caractérisé en ce que** l'unité d'enregistrement (14) réalise en plus de l'enregistrement initial un nouvel enregistrement à au moins un autre moment afin d'augmenter davantage une précision de la corrélation.

5. Système d'assistance chirurgicale (1) selon l'une quelconque des revendications précédentes, **caractérisé en ce que** l'endoscope (2), en particulier une section de manipulation de l'endoscope (2), est guidée par robot, en particulier au moyen d'un bras de robot, et le système de suivi (16) détermine la position et/ou l'orientation et/ou le déplacement de l'endoscope (2), en particulier de la tête d'enregistrement (6), par le biais d'une position et/ou une orientation et/ou un déplacement correspondant du robot, en particulier du bras de robot, et ainsi par le biais du robot.

6. Système d'assistance chirurgicale (1) selon l'une quelconque des revendications précédentes, **caractérisé en ce que** le dispositif de commande (12) est adapté afin d'afficher en position correcte dans la représentation de corrélation (K), dans la représentation de la vue des données d'enregistrement en 3D (3DA) et/ou de l'enregistrement intracorporel (IA) outre l'endoscope (2 ; 2'), des instruments (24') médicaux préplanifiés enregistrés dans l'unité de mémoire (10) et/ou des implants, en particulier un trocart préplanifié.

7. Système d'assistance chirurgicale (1) selon l'une quelconque des revendications précédentes, **caractérisé en ce que** le dispositif de commande (12) est adapté afin d'afficher dans la représentation de corrélation (K), dans la représentation de la vue des données d'enregistrement en 3D (3DA) et/ou de l'enregistrement intracorporel (IA) un trajet planifié et/ou des annotations afin de guider un chirurgien vers le champ opératoire.

8. Système d'assistance chirurgicale (1) selon l'une quelconque des revendications précédentes, **caractérisé en ce que** le dispositif de commande (12) est adapté afin d'établir la vue des données d'enregistrement en 3D (3DA)
comme scène en 3D ou
comme sections transversales bidimensionnelles par rapport à un système de coordonnées d'image de l'endoscope (2) et/ou le long d'un axe d'enregistrement de l'endoscope (2), en particulier de la tête d'enregistrement (6), ou
comme image d'endoscope virtuelle.

9. Support de stockage lisible par ordinateur, comprenant des commandes qui en cas de réalisation par un ordinateur entraînent celui-ci à réaliser les étapes de procédé d'un procédé de représentation d'enregistrement pour la représentation corrélée, centrale de deux enregistrements différents, en particulier pour un système d'assistance chirurgicale (1) selon l'une quelconque des revendications 1 à 8 qui est **caractérisé par** les étapes suivantes :
(51) la lecture de données d'enregistrement en 3D (3DA) numériques, en particulier des données d'enregistrement en 3D (3DA) pré-opérationnelles, d'un patient (P) ;
(S2) l'établissement ou la détection d'un enregistrement intracorporel (IA) par l'endoscope (2) selon la revendication 1-8 ;
(S3) la détection d'au moins un point de repère (15) et/ou d'une orientation anatomique dans l'enregistrement intracorporel (IA) ;
(S4) la détermination, par une unité d'enregistrement (14), d'un point de repère correspondant (15') et/ou d'une orientation dans les données d'enregistrement en 3D (3DA) ;
(S5) l'enregistrement, par l'unité d'enregistrement (14), des données d'enregistrement en 3D (3DA) en l'enregistrement intracorporel (IA) par le biais d'au moins un point de repère (15, 15') correspondant détecté et/ou d'une orientation ;
(S6) la génération, par un dispositif de commande (12), d'une représentation de corrélation (K) avec au moins une représentation de l'enregistrement intracorporel (IA) et au moins une représentation d'une vue de données d'enregistrement en 3D (3DA) ainsi que de préférence l'émission de la représentation de corrélation (K) par le dispositif de représentation ;
(S7) la détection continue, par le système de suivi (16) selon la revendication 1-8, d'une position et/ou orientation et/ou déplacement de l'endoscope (2) selon la revendication 1-8, en particulier d'une tête d'enregistrement (6) ;
(S8) la transmission, par le dispositif de commande (12), du déplacement détecté de l'endoscope (2), en particulier de la tête d'enregistrement (6), à au moins une position et/ou orientation virtuelle d'une tête d'enregistrement virtuelle (6'), en particulier d'un endoscope virtuel (2'), dans la vue des données d'enregistrement en 3D (3DA) et
(S9) la génération, par le dispositif de commande (12), d'au moins une représentation de corrélation (K) actualisée avec l'enregistrement intracorporel (IA) et la vue actualisée des données d'enregistrement en 3D (3DA) et l'émission de la représentation de corrélation (K) par le dispositif de représentation.

10. Support de stockage lisible par ordinateur selon la revendication 9, **caractérisé en ce que** le procédé de représentation d'enregistrement présente de plus les étapes suivantes :
la détermination, à l'aide des points de repère détectés et des points de repère correspondants déterminés, en particulier à l'aide d'une comparaison de surface, d'une déformation d'une structure anatomique réelle, en particulier par rapport à une structure anatomique initiale avec des points de repère initiaux, et
la transmission de cette déformation déterminée à la structure anatomique virtuelle dans les données d'enregistrement en 3D afin d'adapter et de corriger en conséquence les données d'enregistrement en 3D.

11. Salle stérile médicale, telle qu'une salle d'opération (100), **caractérisée en ce que** la salle stérile médicale présente un système d'assistance chirurgicale (1) selon l'une quelconque des revendications 1 à 8.
